(19) Europäisches Patentamt / European Patent Office / Office européen des brevets

(11) **EP 4 190 892 A1**

(12) **EUROPEAN PATENT APPLICATION**
published in accordance with Art. 153(4) EPC

(43) Date of publication:
**07.06.2023 Bulletin 2023/23**

(21) Application number: **21850066.8**

(22) Date of filing: **30.07.2021**

(51) International Patent Classification (IPC):
*C12N 5/07* (2010.01)         *A61K 35/19* (2015.01)
*A61K 35/28* (2015.01)       *A61K 35/30* (2015.01)
*A61K 35/36* (2015.01)       *A61K 35/407* (2015.01)
*A61K 35/545* (2015.01)     *A61P 9/10* (2006.01)
*A61P 43/00* (2006.01)       *C12N 5/0735* (2010.01)
*C12N 5/0775* (2010.01)     *C12N 5/0793* (2010.01)

(52) Cooperative Patent Classification (CPC):
A61K 35/19; A61K 35/28; A61K 35/30;
A61K 35/36; A61K 35/407; A61K 35/545;
A61P 9/10; A61P 43/00; C12N 5/06

(86) International application number:
**PCT/JP2021/028298**

(87) International publication number:
**WO 2022/025238 (03.02.2022 Gazette 2022/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.07.2020  JP 2020130744**

(71) Applicant: **Rainbow Inc.
Sapporo-shi, Hokkaido, 001-0021 (JP)**

(72) Inventor: **KAWABORI, Masahito
Sapporo-shi, Hokkaido 060-0808 (JP)**

(74) Representative: **Engelhard, Markus
Boehmert & Boehmert
Anwaltspartnerschaft mbB
Pettenkoferstrasse 22
80336 München (DE)**

(54) **PLATELET LYSATE DERIVED FROM AUTOLOGOUS PLATELETS**

(57)     The present disclosure provides a method for preparing a platelet lysate for use in the culture of cells collected from an individual. More specifically, the present disclosure provides a method for preparing a platelet lysate for use in the culture of cells collected from an individual, in which the method includes a step for freeze-thawing platelets collected from the individual or an equivalent of the platelets to produce a platelet lysate, and does not include a step for carrying out decomplementation. In some embodiments, the platelets or the equivalent thereof may be freeze-thawed one or several times.

EP 4 190 892 A1

**Description**

[Technical Field]

The present disclosure relates to cell culture using platelet lysates and techniques related thereto.

[Background Art]

Regenerative medicine is drawing attention as a new treatment method for central nervous system diseases (cerebral infarction, cerebral hemorrhage, head injury, Parkinson's disease, etc.), and development of multiple cell medicines is underway. The present inventors have previously developed a treatment by direct transplantation that delivers cells directly into the brain, and have obtained excellent therapeutic results (Non Patent Literature 1).

[Citation List]

[Non Patent Literature]

[NPL 1] Shichinohe, Kawabori et al. Research on advanced intervention using novel bone marrow stem cell (RAINBOW): a study protocol for a phase I, open-label, uncontrolled, dose-response trial of autologous bone marrow stromal cell transplantation in patients with acute ischemic stroke BMC Neurol., Sep 8, 2017; 17 (1) : 179

[Summary of Invention]

[Solution to Problem]

It takes a lot of time and effort to prepare cells to be transplanted, and as a result of intensive studies, the present inventors have found a culture medium, and a culture method, that allows highly efficient culture and is highly safe. Specifically, the present inventors have found that cells can be cultured with high efficiency by using a platelet lysate (PL), which is used as a culture medium, under specific preparation conditions.

The present disclosure provides, for example, the following items.

(Item 1)

A method for preparing a platelet lysate for use in culturing cells obtained from an individual, the method comprising the step of:
freezing and thawing platelets obtained from the individual or equivalents thereof to obtain a platelet lysate, wherein the method does not comprise the step of inactivation.

(Item 2)

A method for culturing cells obtained from an individual, or for culturing cells obtained from an individual to obtain a cell product, the method comprising the steps of:

A) obtaining the cells from an individual;
B) obtaining platelets from the individual or equivalents thereof;
C) freezing and thawing the platelets or the equivalents thereof to obtain a platelet lysate without inactivation; and
D) culturing the cells in the presence of the platelet lysate.

(Item 3)

The method of Item 1 or 2, wherein the platelets or the equivalents thereof are frozen and thawed twice or more.

(Item 4)

The method of Item 3, wherein the platelets or the equivalents thereof are frozen and thawed three times.

(Item 5)

The method of any one of Items 1 to 4, wherein the platelets or the equivalents thereof are frozen at least once at a temperature below -20°C.

(Item 6)

The method of any one of Items 1 to 5, wherein the platelets or the equivalents thereof are frozen at least once at a temperature of -30°C or lower.

(Item 7)

The method of any one of Items 1 to 6, wherein the platelets or the equivalents thereof are frozen at least once at a temperature of -80°C or lower.

(Item 8)

The method of any one of Items 1 to 7, wherein the platelets or the equivalents thereof are frozen at least once at a temperature of -80°C to -196°C.

(Item 9)

The method of any one of Items 1 to 8, wherein the platelets or the equivalents thereof are frozen at least once at a temperature of -80°C.

(Item 10)

The method of any one of Items 1 to 9, wherein the cells are selected from the group consisting of stem cells, nerve cells, corneal cells, epithelial cells, and hepatocytes.

(Item 11)

The method of Item 10, wherein the cells are stem cells.

(Item 12)

The method of Item 11, wherein the stem cells are mesenchymal stem cells.

(Item 13)

The method of Item 12, wherein the mesenchymal stem cells are mesenchymal stem cells derived from bone marrow, adipose tissue, placental tissue, synovial tissue, umbilical cord tissue (*e.g.*, cord blood), dental pulp, or amnion, or mesenchymal stem cells differentiated from ES cells or iPS cells.

(Item 14)

The method of Item 13, wherein the mesenchymal stem cells are bone marrow-derived mesenchymal stem cells.

(Item 15)

The method of any one of Items 1 to 14, wherein the cells are cells that are used in cell injection therapy and drug discovery research and that are obtained from a subject subjected to the cell injection therapy.

(Item 16)

The method of Item 15, wherein the platelets are platelets obtained from the subject.

(Item 17)

A platelet lysate prepared according to the method of any one of Items 1 to 16.

(Item 18)

A formulation comprising a platelet lysate for the culture of cells obtained from an individual, wherein the platelet lysate is a platelet lysate prepared from platelets obtained from the individual or equivalents thereof, and wherein the platelet lysate is not inactivated.

(Item 1A)

A method for culturing cells obtained from an individual, the method comprising the steps of:

A) obtaining the cells from an individual;
B) obtaining platelets from the individual or equivalents thereof;
C) freezing and thawing the platelets or the equivalents thereof to obtain a platelet lysate without inactivation; and
D) culturing the cells in the presence of the platelet lysate.

(Item 2A)

A method for culturing cells obtained from an individual to obtain a cell product, the method comprising the steps of:

A) obtaining the cells from an individual;
B) obtaining platelets or equivalents thereof from the individual;
C) freezing and thawing the platelets or the equivalents thereof to obtain a platelet lysate without inactivation; and
D) culturing the cells in the presence of the platelet lysate and optionally forming the obtained cells into a cell product.

(Item 3A)

The method of Item 1A or 2A, wherein the platelets or the equivalents thereof are frozen and thawed twice or more.

(Item 4A)

The method of Item 3A, wherein the platelets or the equivalents thereof are frozen and thawed three times.

(Item 5A)

The method of any one of Items 1A to 4A, wherein the platelets or the equivalents thereof are frozen at least once at a temperature below -20°C.

(Item 6A)

The method of any one of Items 1A to 5A, wherein the platelets or the equivalents thereof are frozen at least once at a temperature of -30°C or lower.

(Item 7A)

The method of any one of Items 1A to 6A, wherein the platelets or the equivalents thereof are frozen at least once at a temperature of -80°C or lower.

(Item 8A)

The method of any one of Items 1A to 7A, wherein the platelets or the equivalents thereof are frozen at least once at a temperature of -80°C to -196°C.

(Item 9A)

The method of any one of Items 1A to 8A, wherein the platelets or the equivalents thereof are frozen at least once at a temperature of -80°C.

(Item 10A)

The method of any one of Items 1 to 9, wherein the cells are selected from the group consisting of stem cells, nerve cells, corneal cells, epithelial cells, and hepatocytes.

(Item 11A)

The method of Item 10A, wherein the cells are stem cells.

(Item 12A)

The method of Item 11A, wherein the stem cells are mesenchymal stem cells.

(Item 13A)

The method of Item 12A, wherein the mesenchymal stem cells are mesenchymal stem cells derived from bone marrow, adipose tissue, placental tissue, synovial tissue, umbilical cord tissue (*e.g.*, cord blood), dental pulp, or amnion, or mesenchymal stem cells differentiated from ES cells or iPS cells.

(Item 14A)

The method of Item 13A, wherein the mesenchymal stem cells are bone marrow-derived mesenchymal stem cells.

(Item 15A)

The method of any one of Items 1A to 14A, wherein the cells are cells that are used in cell injection therapy and drug discovery research and that are obtained from a subject subjected to the cell injection therapy.

(Item 16A)

The method of Item 15A, wherein the platelets are platelets obtained from the subject.

(Item 1B)

A method of performing cell infusion therapy in an individual in need thereof, the method comprising the steps of:

freezing and thawing platelets obtained from the individual or equivalents thereof to obtain a platelet lysate without inactivation;
culturing cells from the individual in the platelet lysate; and
injecting an effective amount of the cultured cells into the individual.

(Item 2B)

The method of Item 1B, wherein the platelets or the equivalents thereof are frozen and thawed twice or more.

(Item 3B)

The method of Item 1B or 2B, wherein the platelets or the equivalents thereof are frozen and thawed three times.

(Item 4B)

The method of any one of Items 1B to 3B, wherein the platelets or the equivalents thereof are frozen at least once at a temperature below -20°C.

(Item 5B)

The method of any one of Items 1B to 4B, wherein the platelets or the equivalents thereof are frozen at least once at a temperature of -30°C or lower.

(Item 6B)

The method of any one of Items 1 to 6, wherein the platelets or the equivalents thereof are frozen at least once at a temperature of -80°C or lower.

(Item 7B)

The method of any one of Items 1B to 6B, wherein the platelets or the equivalents thereof are frozen at least once at a temperature of -80°C to -196°C.

(Item 8B)

The method of any one of Items 1B to 7B, wherein the platelets or the equivalents thereof are frozen at least once at a temperature of -80°C.

(Item 9B)

The method of any one of Items 1B to 8B, wherein the cells are selected from the group consisting of stem cells, nerve cells, corneal cells, epithelial cells, and hepatocytes.

(Item 10B)

The method of Item 9B, wherein the cells are stem cells.

(Item 11B)

The method of Item 10B, wherein the stem cells are mesenchymal stem cells.

(Item 12B)

The method of Item 11B, wherein the mesenchymal stem cells are mesenchymal stem cells derived from bone marrow, adipose tissue, placental tissue, synovial tissue, umbilical cord tissue (*e.g.*, cord blood), dental pulp, or amnion, or mesenchymal stem cells differentiated from ES cells or iPS cells.

(Item 13B)

The method of Item 12B, wherein the mesenchymal stem cells are bone marrow-derived mesenchymal stem cells.

(Item 14B)

The method of any one of Items 1B to 13B, wherein the cells are cells that are used in cell injection therapy and drug discovery research and that are obtained from a subject subjected to the cell injection therapy.

(Item 15B)

The method of Item 14B, wherein the platelets are platelets obtained from the subject.
In the present disclosure, it is intended that the above one or more features may be provided in further combinations, in addition to the explicit combinations. Still further embodiments and advantages of the present disclosure will be appreciated by those skilled in the art upon reading and understanding the following detailed description as necessary.

[Brief Description of Drawings]

[Figure 1] Figure **1** shows a graph for comparing PDGF values according to the number of times of freezing and thawing. The vertical axis indicates PDGF (mg/ml).

[Figure 2] Figure **2** shows a graph for comparing PDGF values by freezing temperature. The vertical axis indicates relative values to "thawed only".

[Figure 3] Figure **3** shows a graph for comparing BDNF values by the number of times of freezing and thawing. The vertical axis indicates BDNF (mg/ml).

[Figure 4] Figure **4** shows a graph for comparing BDNF values by freezing temperature. The vertical axis indicates relative values to "thawed only".

[Figure 5] Figure **5** shows a graph for comparing TGF-$\beta$1 values by the number of times of freezing and thawing. The vertical axis indicates TGF-$\beta$1 (mg/ml).

[Figure 6] Figure **6** shows a graph for comparing TGF-$\beta$1 values by freezing temperature. The vertical axis indicates relative values to "thawed only".

[Figure 7] Figure **7** shows a graph for comparing PDGF expression levels with and without inactivation. The vertical axis indicates relative values to the case with inactivation.

[Figure 8] Figure **8** shows a graph for comparing BDNF expression levels with and without inactivation. The vertical axis indicates relative values to the case with inactivation.

[Figure 9] Figure **9** shows a graph for comparing TGF-$\beta$1 expression levels with and without inactivation. The vertical axis indicates relative values to the case with inactivation.

[Figure 10] Figure **10** shows a summary of the results of Example 3.

[Figure 11] Figure **11** shows the results of HGF measurement, HGF measurement/cell number, $\beta$-NGF measurement, and $\beta$-NGF measurement/cell number, by the allogeneic conventional method and the new autologous -80°C method in Example 4.

[Figure 12] Figure **12** shows a graph of the number of cells after 4 days and 7 days of culture by the allogeneic conventional method and the new autologous -80°C method in Example 4.

[Figure 13] Figure **13** shows observation images of cells cultured in Example 5.

[Figure 14] Figure **14** shows a graph comparing the amount of PDGF-BB by different freezing temperatures. Each bar in the results of 90 minutes, 120 minutes, 180 minutes and 240 minutes indicates -10°C, -20°C, -30°C, -80°C and -196°C from the left.

[Figure 15] Figure **15** shows a graph comparing the amount of BDNF by different freezing temperatures. Each bar in the results of 90 minutes, 120 minutes, 180 minutes and 240 minutes indicates -10°C, -20°C, -30°C, -80°C and -196°C from the left.

[Figure 16] Figure **16** shows a graph comparing the amount of TGF-$\beta$1 by different freezing temperatures. Each bar in the results of 90 minutes, 120 minutes, 180 minutes and 240 minutes indicates -10°C, -20°C, -30°C, -80°C and -196°C from the left.

[Figure 17] Figure **17** shows a graph comparing the amount of PDGF-BB, T-BDNF and TGF-$\beta$1 by different thawing temperatures.

[Figure 18] Figure **18** shows a graph comparing the amount of PDGF-BB by different thawing temperatures.

[Figure 19] Figure **19** shows a graph comparing the amount of T-BDNF by different thawing temperatures.

[Figure 20] Figure **20** shows a graph comparing the amount of TGF-$\beta$1 by different thawing temperatures.

[Figure 21] Figure **21** shows a graph comparing the amount of growth factor by the PL production method.

[Figure 22] Figure **22** shows observation images of cultured cells.

[Figure 23] Figure **23** shows a graph comparing growth curves with different thawing temperatures.

[Figure 24] Figure **24** shows a graph comparing the amount of PDGF-BB by the number of times of freezing and thawing.

[Figure 25] Figure **25** shows microscopic images of adipocytes, osteocytes and chondrocytes differentiated from mesenchymal stem cells.

[Figure 26] Figure **26** shows a graph of the amount of exosomes per cell in mesenchymal stem cells cultured with the platelet lysate of the present disclosure.

[Figure 27] Figure **27** shows FACS results of mesenchymal stem cells cultured with the platelet lysate of the present disclosure for CD73, CD90, CD105, CD34, CD14, HLA-DR, CD79a, CD19, CD45 and CD11b.

[Description of Embodiments]

Hereinafter, the present disclosure will be described. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence. As used herein, "about" means $\pm$ 10% of the value that follows.

(Definitions)

As used herein, the term "individual" refers to any individual biological entity, and genetically equivalent individuals, even if separate, are considered identical individuals for the purposes of this disclosure.

As used herein, the term "cells obtained from an individual" includes so-called primary cells as well as cells obtained by subculturing primary cells.

As used herein, the term "platelets obtained from an individual" includes so-called primary cell platelets as well as equivalents thereof, *i.e.*, platelets genetically equivalent to the individual, such as platelets obtained by subculturing primary cell platelets, platelets obtained by induction from progenitor cells obtained from the individual, or platelets derived from iPS cells generated from the individual.

As used herein, the term "platelet lysate" refers to a combination of growth factors contained in platelets released by lysing the platelets. This can be obtained by chemical means (*e.g.,* $CaCl_2$), osmotic means (*e.g.,* use of distilled $H_2O$), ultrasonic crushing, transmembrane shear, or freezing and thawing.

As used herein, the term "inactivation" refers to an operation performed to deactivate complement components in serum, and is achieved, for example, by heat treatment (*e.g.,* at about 58°C for 1 hour).

As used herein, the term "mesenchymal stem cell" is also referred to as a mesenchymal stromal cell, and refers to a stem cell that have the ability to differentiate into cells belonging to the mesenchymal system, such as osteoblasts, adipocytes, muscle cells, and chondrocytes. The mesenchymal stem cells may encompass mesenchymal stem cells derived from bone marrow, adipose tissue, placental tissue, synovial tissue, umbilical cord tissue (*e.g.,* cord blood), dental pulp, or amnion, or mesenchymal stem cells differentiated from ES cells or iPS cells.

As used herein, the term "freezing and thawing" is synonymous with the term "freezing and melting" and refers to freezing and then thawing or melting material such as cells. The freezing and thawing or melting is detailed elsewhere herein.

As used herein, the term "sample" refers to a substance or organism used as a subject, and may include platelets and the like.

As used herein, the term "Ready-to-Use formulation" refers to a formulation that can be used as it is without further culturing of cells after preservation.

(Preferred Embodiments)

The preferred embodiments of the present disclosure are described hereinafter. It is understood that the embodiments are an exemplification of the present disclosure, so that the scope of the present invention is not limited to such preferred embodiments. It should be understood that those skilled in the art can refer to the following preferred embodiments to readily make modifications within the scope of the present disclosure. These embodiments of the present disclosure can be appropriately combined with any embodiment by those skilled in the art.

(Method for Preparing Platelet Lysate)

In one aspect, the present disclosure provides a method for preparing a platelet lysate for use in culturing cells obtained from an individual, the method comprising the step of freezing and thawing platelets obtained from the individual or equivalents thereof to obtain a platelet lysate, where the method does not comprise the step of inactivation.

In another aspect, the present disclosure provides a method for culturing cells obtained from an individual, or a method for culturing cells obtained from an individual to obtain a cell product, the method comprising: A) obtaining the cells from an individual; B) obtaining platelets from the individual or equivalents thereof; C) freezing and thawing the platelets or the equivalents thereof to obtain a platelet lysate without inactivation; and D) culturing the cells in the presence of the platelet lysate.

In some embodiments, the method may further include the step of forming cultured cells into a cell product. In some embodiments, the method may further include the step of storing the resulting cell product in a frozen or non-frozen state until use. The resulting cell product may be a readily usable (ready-to-use) formulation. The step of forming cultured cells into a cell product may include housing the cultured cells in a container with a preservative solution.

In some embodiments, examples of containers in which cells are housed include, but are not limited to, tubes, vials, syringes, dishes, plates (12-, 24-, 48- or 96-well plates), and the like.

In some embodiments, platelets or equivalents thereof may be frozen and thawed one or more times. In order to extract more platelet trophic factors, it is preferable to freeze and thaw such platelets or equivalents multiple times, and they can be frozen and thawed twice or more, for example, 2, 3, 4, 5, 6, or more times. In certain embodiments, platelets or equivalents thereof can be frozen and thawed three times.

In some embodiments, platelets or equivalents thereof may be frozen at least once at a temperature of about -20°C or below. In some embodiments, platelets or equivalents thereof may be frozen at least once at a temperature of about -20°C or less, about -30°C or less, about -40°C or less, about -50°C or less, about -60°C or less, about -70°C or less, or about -80°C or less. When frozen multiple times, they may be frozen at different temperatures or at the same temperature. In certain embodiments, samples including platelets may be frozen at temperatures from about -80°C to about -196°C. If the samples are frozen at about -196°C, they may be frozen using liquid nitrogen. As used herein, the freezing in liquid nitrogen means freezing at about - 196°C. In certain embodiments, the platelets or equivalents thereof may be frozen at a temperature of -80°C at least once.

Platelet lysates prepared by the methods of the present disclosure are used to culture any cell. Examples of cells to be cultured include, but are not limited to, stem cells, nerve cells, corneal cells, epithelial cells, hepatocytes, and the like. In some embodiments, the cells may be stem cells. In certain embodiments, stem cells may be mesenchymal stem cells (mesenchymal stromal cells). In further certain embodiments, the mesenchymal stem cells may be mesenchymal stem cells derived from bone marrow, adipose tissue, placental tissue, synovial tissue, umbilical cord tissue (*e.g.,* cord blood), dental pulp, or amnion, or mesenchymal stem cells differentiated from ES cells or iPS cells. In a preferred embodiment, the cells may be bone marrow-derived mesenchymal stem cells.

These cells can be cells used in cell infusion therapy and drug discovery research. In cell injection therapy, the cells may be cells obtained from a subject or cells obtained from a different subject. The platelets may be platelets obtained from a subject or platelets obtained from a different subject. The cells to be cultured and the platelets may be of the same origin (*i.e.,* autologous) or different origins (*i.e.,* allogeneic). In a preferred embodiment, the platelets may be autologous.

The platelet lysate of the present disclosure may be used to differentiate iPS cells into specific cell groups (such as bone) and efficiently culture them (such as in iPS drug discovery research).

(Platelet Lysates and Formulations)

In one aspect, the present disclosure provides a platelet lysate prepared according to the above method. The platelet lysate of the present disclosure is capable of culturing cells with high efficiency and is therefore advantageous.

In a further aspect, the present disclosure provides a formulation including a platelet lysate for culturing cells obtained from an individual, in which the platelet lysate is a platelet lysate prepared from platelets obtained from the individual or equivalents thereof, and in which the platelet lysate is not inactivated.

(Cell Culturing and Cell Product Manufacturing)

In another aspect, the present disclosure provides a method for culturing cells obtained from an individual, the method comprising the steps of: A) obtaining the cells from an individual; B) obtaining platelets from the individual or equivalents thereof; C) freezing and thawing the platelets to obtain a platelet lysate without inactivation; and D) culturing the cells in the presence of the platelet lysate.

Cells or cell culture obtained by the technique of the present disclosure exhibit remarkable effects as compared to conventional techniques.

For example, in cell proliferation, the presence of platelet lysate of the present disclosure results in an about 2 to about 40-fold, about 2 to about 20-fold, or about 5 to about 20-fold improvement, such as an about 2-fold, about 3-fold, about 4-fold, about 5-fold, about 6-fold, about 7-fold, about 8-fold, about 9-fold, about 10-fold, about 12-fold, about 14-fold, about 16-fold, about 18-fold, or about 20-fold improvement, in proliferation efficiency (time required to reach the desired number of cells) compared to methods that do not use platelet lysate, for example, the presence of bovine serum (Fetal bovine serum, 10%); and the presence of platelet lysate of the present disclosure results in an about 20 to about 200-fold, about 20 to about 100-fold, or about 50 to about 100-fold improvement, such as an about 20-fold, about 30-fold, about 40-fold, about 50-fold, about 60-fold, about 70-fold, about 80-fold, about 90-fold, or about 100-fold improvement, in proliferation efficiency (time required to reach the desired number of cells) compared to no addition of bovine serum and no addition of platelet lysate (Rauch et al., ALTEX. 2011; 28(4): 305-16. doi: 10.14573/altex.2011.4.305.). Further, with regard to the presence of the platelet lysate of the present disclosure, the presence results in an about 2 to about 20-fold, about 2 to about 10-fold, or about 5 to about 10-fold improvement, such as an about 2-fold, about 3-fold, about 4-fold, about 5-fold, about 6-fold, about 7-fold, about 8-fold, about 9-fold, or about 10-fold improvement, in proliferation efficiency (time required to reach the desired number of cells) compared to conventional platelet lysates (such as those of allogeneic platelet origin, with inactivation, and frozen and thawed only once).

The obtained cells have significantly improved culture efficiency although the obtained cells ensure the same quality as cells cultured with conventional platelet lysate, and are therefore advantageous.

In the methods of the present disclosure, the desired number of cells can be from about 2 million to about 2 billion. This is because about 2 million to about 2 billion cells are sufficient for cell injection for treatment of, for example, cerebral infarction. In a preferred embodiment, the desired number of cells may be from about 20 million to about 200 million.

In another aspect, the present disclosure provides a method for culturing cells obtained from an individual to obtain a cell product, the method comprising the steps of: A) obtaining the cells from an individual; B) obtaining platelets or equivalents thereof from the individual; C) freezing and thawing the platelets to obtain a platelet lysate without inactivation; and D) culturing the cells in the presence of the platelet lysate and optionally forming the obtained cells into a cell product.

In the manufacture of cell products, a reduction in product manufacturing time of at least about 10%, at least about 15%, at least about 20%, at least about 25%, at least about 30%, at least about 35%, at least about 40%, at least about 45%, or at least about 50% is seen compared to the use of conventional platelet lysates (such as those of allogeneic platelet origin, with inactivation, and frozen and thawed only once). For example, according to the results of Example 3, the platelet lysate produced by freezing and thawing three times without inactivation yielded 8 times more cells than the platelet lysate produced by freezing and thawing once with inactivation. Assuming that the cell division rate is 36 hours (the division cycle of bone marrow stem cells is approximately 24 to 48 hours), this means that the platelet lysate allows cells to proliferate in 16 days when it takes, for example, 21 days to reach a given number of cells; and this results in a 23% reduction in culture time. Further, in the manufacture of cell products, the presence results in an about 2 to about 20-fold, about 2 to about 10-fold, or about 5 to about 10-fold improvement, such as an about 2-fold, about 3-fold, about 4-fold, about 5-fold, about 6-fold, about 7-fold, about 8-fold, about 9-fold, or about 10-fold improvement, in proliferation efficiency (time required to reach the desired number of cells) compared to conventional platelet lysates (such as those of allogeneic platelet origin, with inactivation, and frozen and thawed once).

The resulting cell products have significantly improved culture efficiency although the cell products ensure the same quality as cells cultured with conventional platelet lysate, and are therefore advantageous.

In some embodiments, platelet lysates can typically be prepared as below. Those skilled in the art can appropriately change conditions such as the amount of reagents used, freezing/thawing temperature, and number of times.

1. Confirm negative for parvovirus at the test 70 days after the blood collection from the donor of the platelet concentrate to be used;

2. Draw out the frozen platelet concentrate for 3 people from the ultra-low temperature freezer and bring it into a preparation room via a pass box;

3. Remove the freezer bag from the canister and leave it to stand in the $CO_2$ incubator;

4. Completely thaw, occasionally turning the freezer bag over and repositioning it;

5. Insert an operating adapter into the port of the freezer bag in a biohazard control cabinet;

6. Draw out the thawed platelet concentrate with a 30 ml syringe (with lock) attached to a 18G injection needle, and dispense it into an even number of 225ml centrifuge tubes;

7. Add 1 ml of heparin per 40 ml of platelets using a 10 ml syringe with a needle in the centrifuge tube from Step 5, and mix well with a 50 ml pipette;

8. Centrifuge at BART = 6,2000×g, 20 minutes, 20°C., accelerator fast, and brake slow;

9. Collect the supernatant with a 50ml pipette into new 225 ml centrifuge tubes (even number);

10. Centrifuge at BART = 6,2000×g, 20 minutes, 20°C., accelerator fast, and brake slow;

11. Collect the supernatant with a 50ml pipette into new 50ml centrifuge tubes (even number);

12. Place the centrifuge tubes from Step 10 in the heat block of an e-thermostatic bath heated to 58°C and leave them to stand for 1 hour to inactivate;

13. Centrifuge at BART = 2,500×g, 5 minutes, 20°C., accelerator fast, and brake slow;

14. Collect the supernatant with a 50ml pipette into new 225ml centrifuge tubes (even number);

15. Centrifuge at BART = 6,500×g, 5 minutes, 20°C., accelerator fast, and brake slow;

16. Combine the supernatant into a 1000 ml bottle with a 50 ml pipette and mix well;

17. Draw out about 7 ml with a 10 ml pipette into a 50 ml centrifuge tube and assign a predetermined lot number. Draw out about 2 ml (for ELISA) into a 5 ml tube and assign a PL serial number; and

18. Dispense 45 ml into each 50 ml centrifugal tube, attach a label to each tube, and store in the freezer part of a medicinal cool box with a freezer.

In some embodiments, cell culture solution can typically be prepared as below. Those skilled in the art can appropriately change conditions such as the amount of reagents used, freezing/thawing temperature, and number of times.

1. Thaw the PL in an e-thermostatic bath at 37°C;
2. Add 0.5 ml of antibiotic to 1 bottle of α-MEM (500 ml) using a syringe with a 1 ml needle;
3. Add 50 ml of PL to 1 bottle of α-MEM at Step 2 with a 50 ml pipette;
4. Connect the filter unit and the suction pump with a suction tube;
5. Pour the culture solution from Step 3 onto the top of the filter unit with a 50 ml pipette and activate the suction pump;
6. When all the poured culture solution has passed through the filter, pour new culture solution;
7. If the suction speed drops significantly, stop the pump once, remove the suction tube from the filter, and change the bottle top filter with a new one;
8. When approximately 500 ml of the filtered culture solution accumulates in the storage bottle at the bottom of the filter unit, stop the pump and remove the suction tube from the filter;
9. Change the bottle top filter with a storage cap;
10. Repeat Steps 4 to 9 until all the culture solution from Step 3 is filtered;
11. Affix a label with a culture solution number on the bottle containing the filtered culture solution; and
12. Draw out approximately 5 ml (for external dispensing) from each bottle of the culture solution, place in a 50 ml centrifuge tube, and assign a predetermined lot number.

In some embodiments, cells may be harvested from an individual's bone marrow fluid. The cells from bone marrow fluid can typically be harvested as below. Those skilled in the art can appropriately change conditions such as the amount of reagents used, freezing/thawing temperature, and number of times.

1. Confirm the results of the visual inspection and donor screening of the acceptance test;
2. If the acceptance standard is met, bring it to the CPC;
3. Check if the isolator is in production, and if not, put it into production according to the isolator manual;
4. Perform the subsequent operations inside the isolator, and carry out loading and unloading of raw materials, reagents, and materials to and from the isolator according to the isolator manual;
5. Check the total amount of bone marrow fluid (1) (to the first place of decimals);
6. Put all the bone marrow fluid into a 225ml centrifuge tube and mix well with a pipette;
7. Draw out 7 ml of specimen for acceptance test into a 50 ml tube (2), and draw out 0.5 ml each of 1 ml of specimen for storage into two cryotubes (3) and (4);
8. Affix a specimen number label on the acceptance test specimen tube, and affix a specimen number/manufacturing number label on the tube for the storage specimen;
9. Hand over the specimen for acceptance testing to quality personnel;
10. Determine the number of tubes into which the gravity-separated liquid is to be dispensed according to the following formula:

```
Quantity of specimen drawn out (5) = (2) + (3) + (4)

Number of dispensing (6) = (total amount of bone marrow
fluid (1) - (5)) × 2 ÷ 20

Number of dispensing (7) = (total amount of bone marrow
fluid (1) - (5)) × 2 ÷ 25
```

Number of dispensing (tubes): integer between (6) and (7);
11. Using a 10-ml pipette, dispense 15 ml of the gravity separated solution to the number of Leukoseps determined in Step 10;
12. Centrifuge at BART = 3,970×g, 30 seconds, 22°C., accelerator fast, and brake fast;
13. Calculate the amount of cell culture solution and heparin for diluting the bone marrow fluid according to the following formula:

$$\text{Cell culture solution (ml)} = ((1) - (5)) \times 0.9$$

$$\text{Heparin (ml)} = ((1) - (5)) \times 0.1;$$

14. Add the amount of cell culture solution (using a 25ml pipette) and heparin (using a 10ml needle syringe) obtained at Step 13 to a 200 ml centrifuge tube;

15. Mix the bone marrow diluent well with a 25 ml pipette and put 20 to 25 ml each into the Leukosep containing the gravity-separated liquid;

16. Centrifuge at BART = $3,970 \times g$, 10 minutes, 22°C., accelerator fast, and brake slow;

17. Remove the upper plasma portion using a 10 ml pipette, leaving about 5 mm above the middle mononuclear cell layer;

18. Collect approximately 20 ml each of the mononuclear cell layer into 50 ml centrifuge tubes with a 10 ml pipette;

19. Add the same amount of physiological saline to the collected mononuclear cells with a 10 ml pipette;

20. Centrifuge at BART = $3,800 \times g$, 5 minutes, 15°C., accelerator fast, and brake slow;

21. Gently remove the supernatant using a 25ml pipette;

22. Add physiological saline to pellets, followed by suspending, and put them together in one centrifugation tube;

23. Centrifuge at BART = $3,800 \times g$, 5 minutes, 15°C., accelerator fast, and brake slow;

24. Gently remove the supernatant using a 25ml pipette;

25. Add 10 ml of cell culture solution to the pellets, followed by suspending, and check the total liquid volume;

26. Take about 0.1 ml from the cell suspension into a 5 ml tube and put the specimen in a zipper bag, then contact the quality personnel to hand over the specimen from the shipping pass box;

28. Obtain test results from quality personnel (total number of cells (8));

29. Determine the number of flasks to seed according to the formula below, but make sure there are at least two:

$$\text{Number of flasks (9)} = \text{total number of cells (8)} / 0.5 \times 10^8$$

$$\text{Number of flasks (10)} = \text{total number of cells (8)} / 2 \times 10^8$$

Number of flasks for seeding: the larger of an integer between (9) and (10) or 2;

30. Add the cell culture solution with a 25 ml pipette so that the cell suspension has the volume calculated by the following formula:

$$\text{Amount of culture solution added} = (\text{number of 175 cm}^2 \text{ flasks} \times 5) - \text{cell volume};$$

31. Put 20 ml each of cell culture solution into 175 cm$^2$ flasks with a 25 ml pipette;

32. Add 5 ml each of the cell suspension from Step 30 to 175 cm$^2$ flasks with a 10 ml pipette;

33. Mix well by tilting the flasks so that the cells are evenly distributed; and

34. Place the flasks in a CO$^2$ incubator and culture at $37 \pm 1$°C and carbon dioxide gas concentration of $5 \pm 1$%.

In some embodiments, subculturing of cells can typically be performed as below. Those skilled in the art can appropriately change conditions such as the amount of reagents used, freezing/thawing temperature, and number of times.

1. Check if the isolator is in production, and if not, put it into production according to the isolator manual;

2. Perform the subsequent operations inside the isolator, and carry out loading and unloading of raw materials, reagents, and materials to and from the isolator according to the isolator manual;

3. Remove the flasks from the CO$_2$ incubator;

4. Observe and photograph at least one flask with the observation module;

5. Discard the culture solution in the flask with a 10 ml pipette for the 175 cm$^2$ flask and with a decanter for the five-layered flask;

6. Add the following amount of TrypLE Select with a 10 ml pipette and spread it over the bottom of the flask

4 ml to the 175cm$^2$ flask
20 ml to the five-layered flask;

7. Leave the flasks to stand in the $CO_2$ incubator for 5 minutes;
8. Sufficiently remove adherent cells by tapping the side of the flask or the like;
9. Collect TrypLE Select into a 50 ml centrifuge tube with a 10 ml pipette;
10. Add the following amount of physiological saline with a

10 ml pipette
4 ml to the 175 cm$^2$ flask
20 ml to the five-layered flask;

11. Rinse the bottom of the flask well by pipetting and collect it in the 50 ml centrifuge tube of Step 9;
12. Centrifuge at BART = 3,800$\times$g, 5 minutes, 15°C., accelerator fast, and brake slow;
13. Gently remove the supernatant using a 10 ml pipette;
14. Add 10 ml physiological saline to pellets, followed by suspending, using a 10 ml pipette;
15. Centrifuge at BART = 3,800$\times$g, 5 minutes, 15°C., accelerator fast, and brake slow.
16. Gently remove the supernatant using a 10 ml pipette;
17. Add 10 ml of culture solution to pellets, followed by suspending, using a 10 ml pipette;
18. Take approximately 0.1 ml from the cell suspension into a 5 ml tube.
19. Contact the quality personnel and hand over the specimen from the shipping pass box;
20. Obtain test results from quality personnel (total number of cells (1));
21. Determine the number of flasks to seed according to the formula below:

$$\text{Number of faces (2)} = \text{total number of cells (1)} / 5 \times 10^5$$

$$\text{Number of 5-layered flasks for calculation (3)} = ((2) - \text{number of 175 cm}^2 \text{ flasks}) / 5$$

$$\text{Number of 5-layered flasks for seeding (4)} = \text{round off (3)}$$

*Seed at least one 175 cm$^2$ flask for observation and two more for in-process control testing at the final passage (three in total);
22. Add the cell culture solution with a 10ml pipette so that the cell suspension has the liquid volume calculated by the following formula:

$$\text{Amount of culture solution added} = (\text{number of 175 cm}^2 \text{ flasks} + \text{number of 5-layered flasks} \times 5) - \text{cell volume};$$

23. Using a 50 ml pipette, pour 24 ml each of cell culture solution into 175 cm$^2$ flasks and 120 ml each into 5-layered flasks;
24. Using a 10ml pipette, add 1ml each of the cell suspension from Step 22 to the 175cm$^2$ flasks and 5ml each to the 5-layered flasks;
25. Mix well by tilting the flasks so that the cells are evenly distributed; and
26. Place the flasks in a $CO_2$ incubator and culture at 37$\pm$1°C and carbon dioxide gas concentration of 5$\pm$1%.

In some embodiments, harvesting and washing cultured cells can typically be performed as below. Those skilled in the art can appropriately change conditions such as the amount of reagents used, freezing/thawing temperature, and number of times.

1. Check if the isolator is in production, and if not, put it into production according to the isolator manual;
2. Perform the subsequent operations inside the isolator, and carry out loading and unloading of raw materials, reagents, and materials to and from the isolator according to the isolator manual;
3. Remove the flasks from the $CO_2$ incubator;

4. Observe and photograph at least one flask with the observation module;

5. Discard the culture solution in the flask with a 10 ml pipette for the 175 $cm^2$ flask and with a decanter for the five-layered flask;

6. Add the following amount of TrypLE Select with a 10ml pipette and spread it over the bottom of the flask

4 ml to the 175$cm^2$ flask
20 ml to the five-layered flask;

7. Leave the flasks to stand in the $CO_2$ incubator for 5 minutes;

8. Sufficiently remove adherent cells by tapping the side of the flask or the like;

9. Collect TrypLE Select into a 50 ml centrifuge tube with a 10 ml pipette;

10. Add the following amount of physiological saline with a 10 ml pipette

4 ml to the 175 $cm^2$ flask
20 ml to the five-layered flask;

11. Rinse the bottom of the flask well by pipetting and collect it in the 50 ml centrifuge tube of Step 9;

12. Centrifuge at BART = 3,800×g, 5 minutes, 15°C., accelerator fast, and brake slow;

13. Gently remove the supernatant using a 10 ml pipette;

14. Suspend pellets in 30 ml physiological saline using a 10 ml pipette and put them together in one tube;

15. Centrifuge at BART = 3,800×g, 5 minutes, 15°C., accelerator fast, and brake slow.

16. During centrifugation, draw out ARTCEREB with a 10ml syringe with a needle and transfer it to a 50ml centrifuge tube.

17. Gently remove the supernatant using a 10ml pipette into a new 50ml tube;

18. Add 4ml of ARTCEREB to the pellets with a 10ml pipette and suspend;

19. Take about 0.1 ml from the cell suspension into a 5 ml tube and label it as a specimen for standard test (cell count/viability);

20. Draw out the specimens below from the supernatant of Step 17:

- 5 ml each for three 50 ml tubes (a, b, c)
- 5.3 ml for a 50 ml tube (d)
- 1 ml each for two cryotubes (e, f);

21. Label each tube in Step 20;

22. Place the specimen of Step 19 and the specimens a, b, and c of Step 21 in separate zippered bags, and hand them over from the shipping pass box to the quality personnel;

a: for sterility test in rapid quality test
b: for endotoxin test in rapid quality test
c: for sterility and endotoxin test in standard test

23. Obtain test results from quality personnel (cell concentration (1));

24. Calculate the cell volume to add to the tube d:

```
test liquid volume (µl) = (5.3 × 10⁵ / cell concentration
(1)) × 1000
```

25. Using a micropipette, draw out the volume of cell suspension calculated in Step 24 and add it to the tube d in Step 21;

26. Draw out 100 pl each from the tube d into two cryotubes, and label them as specimens for storage, where the rest is used for the standard test (mycoplasma negative test);

27. Calculate the cell volume required for administration (2) :

```
high dose group: cell volume (ml) = 5 × 10⁷ / cell
concentration (1)
```

```
low   dose   group:   cell   volume   (ml)   =   2   ×   10⁷   /   cell
concentration (1);
```

28. Draw out the volume of cell suspension calculated in Step 27 into a 5 ml tube, using a micropipette.
29. Centrifuge at BART = 5,800×g, 5 minutes, 15°C., accelerator fast, and brake slow;
30. Calculate the supernatant removal volume so that the final volume is 1 ml for the high dose group and 0.4 ml for the low dose group:

```
high dose group (μL) = (suspension volume (2) - 1.0) × 1000

low dose group (μL) = (suspension volume (2) - 0.4) × 1000;
```

31. Remove the volume of supernatant calculated in Step 30 with a micropipette;
32. Thoroughly suspend the pellets;
33. Hand over to the visual inspection personnel;
34. Place the specimen for the standard test (mycoplasma negative test) in Step 26 in a zipper bag and hand it over from the shipping pass box to the quality personnel; and
35. Store the tubes e and f of Step 21 and the two cryotubes of Step 26 in a -150°C freezer in the cell storage room as specimens for storage.

(Confirmation of Gene Expression Profile of Cultured Cells)

In some embodiments, genetic profiles in cultured cells may be confirmed. In certain embodiments, the cells can be stem cells (such as bone marrow-derived mesenchymal stem cells), and genetic profiles can be confirmed, for example, according to Example 4. In some embodiments, in the cultured cells, expression of genes associated with one or more selected from the group consisting of the following categories, for example, may be enhanced:

1. Protein localization to cytoskeleton
2. Regulation of mitotic metaphase/anaphase transition
3. Cell cycle checkpoint
4. Chromosome segregation
5. Extracellular matrix organization
6. Wound healing
7. Cell division
8. Mitotic Cell Cycle process
9. Tissue development
10. Animal organ development

Preferably, the expression of genes associated with one or more selected from the group consisting of the above 2, 3 and 7, which are associated with cell division, and the above 6, which is associated with tissue repair, may be enhanced.

In some embodiments, in cultured cells, expression of genes associated with one or more selected from the group consisting of the following categories may be decreased:

1. Histone dephosphorylation
2. Very-low-density lipoprotein particle clearance
3. regulation of cardioblast proliferation
4. Chylomicron remnant clearance
5. pharyngeal system development
6. artery morphogenesis
7. regulation of bone mineralization
8. Response to cAMP
9. negative regulation of small molecule metabolic process
10. Regulation of receptor signaling pathway via JAK-STAT
11. Negative regulation of neuron apoptotic process

12. Extracellular matrix organization
13. Positive regulation of peptidyl-tyrosine phosphorylation
14. Response to reactive oxygen species
15. Positive regulation of stress-activated MAPK cascade
16. Positive regulation of protein kinase B signaling
17. Regulation of JNK cascade
18. Striated muscle tissue development
19. Regulation of epithelial cell proliferation
20. Regulation of ERK1 and ERK2 cascade
21. Regulation of MAP kinase activity
22. Positive regulation of protein serine/threonine kinase activity
23. Response to acid chemical
24. Muscle structure development
25. Cytokine-mediated signaling pathway
26. Cellular response to hormone stimulus
27. Response to lipid
28. Cellular response to growth factor stimulus
29. Enzyme linked receptor protein signaling pathway
30. Regulation of cell adhesion
31. Positive regulation of cell differentiation
32. Positive regulation of cell death
33. Regulation of cell migration
32. Anatomical structure formation involved in morphogenesis
33. Positive regulation of cell population proliferation
34. Cellular response to oxygen-containing compound
35. Animal organ morphogenesis
36. Cell migration
37. Positive regulation of multicellular organismal process
38. Regulation of cell cycle
39. Positive regulation of transcription, DNA-templated
40. Regulation of immune system process
41. Cell differentiation

Preferably, the expression of genes associated with one or more selected from the group consisting of the above 6, 7, 18, 19, 24, 32, 37 and 41, which are associated with promotion of cell differentiation, and the above 32, which is associated with promotion of cell death, may be decreased.

(Treatment Methods and Treatment Techniques)

In another aspect, the present disclosure provides a method of performing cell infusion therapy in an individual in need thereof, the method comprising the steps of: freezing and thawing platelets obtained from the individual or equivalents thereof to obtain a platelet lysate; culturing cells from the individual with the platelet lysate without inactivation; and injecting an effective amount of the cultured cells into the individual.

(Use in Treatment of Cerebral Infarction)

Typically, prepared cells can be used in stroke patients as follows.

(1) Patients as Subjects

Subjects are patients in the acute phase (early onset) of cerebral infarction who meet the following five criteria: those aged 20 or over and under 80 years old at the time of informed consent; those within 14 days after the onset of cerebral infarction at the time of informed consent; those with cerebral infarction occurring in the internal carotid artery perfusion area; those whose mRS before onset of cerebral infarction is 0 or 1; those with moderate to severe neurological symptoms due to cerebral infarction (NIHSS (National Institutes of Health Stroke Scale): $\geq 6$) (however, a total of 6 points or more is required in the categories "5. Upper Extremity Movement" and "6. Lower Extremity Movement" of NIHSS), who do not meet the prescribed exclusion criteria, and who give consent to this study.

(2) Preparation and Administration of Bone Marrow Stem Cells

After patient registration, the patient's bone marrow was promptly collected, and cell culture and preparation of bone marrow stem cells were performed at the Cell Processing Laboratory, Clinical Research and Medical Innovation Center, Hokkaido University Hospital. Prepared bone marrow stem cells (20 million or 50 million/patient) are administered directly into the patient's brain three to five weeks after bone marrow harvest.

As described above, the present disclosure has been described while showing preferred embodiments to facilitate understanding. The present disclosure is described hereinafter based on Examples. The above descriptions and the following Examples are not provided to limit the present disclosure, but for the sole purpose of exemplification. Thus, the scope of the present disclosure is not limited to the embodiments or the Examples specifically described herein and is limited only by the scope of claims.

[Examples]

The present disclosure will be described hereinafter in further detail with Examples.

(Example 1: Number of Times of Freezing and Thawing and Difference in Extraction of Trophic Factor)

(Materials and Methods)

A concentrated platelet solution (made by JRCS) collected in a liquid state was placed in freezing bags (Nipro Froze Bag F-100; Catalog No. 89-100, 50 ml) and stored at -80°C. The concentrated platelet solution was then thawed overnight in a refrigerator at 4°C ("thawed only"). This was frozen overnight at -20°C or -80°C and thawed again at 4°C ("thawed + frozen once"). This was frozen overnight at -20°C or -80°C and thawed again at 4°C ("thawed + frozen once").

Those subjected to the same procedure are indicated as "thawed + frozen twice", and those further frozen and thawed are indicated as "thawed + frozen three times". The obtained platelet lysate (10 ml) was mixed with 0.25 ml of heparin, centrifuged several times, mixed with 100 ml of MEM$\alpha$, and passed through a filter (0.45 $\mu$m). ELISA was then performed.

(Results)

The results without distinction between -20°C and -80°C are shown in (A/B/C-1), and the results with comparison between -20°C and -80°C are shown in (A/B/C-2) below.

(A-1) Platelet-Derived Growth Factor (PDGF)

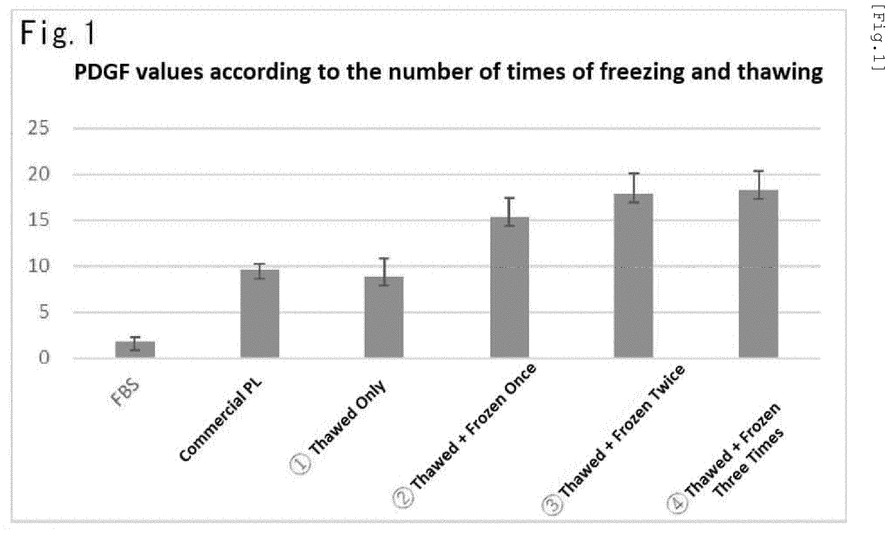

Table 1 summarizes the amount of platelet-derived growth factor (PDGF) in each sample. Figure **1** is a graphical representation of Table 1. As shown in Table 1, repeated freezing and thawing increased PDGF in solution. FBS contained very little PDGF. Commercially available PL and "thawed only" were comparable. Frozen samples contained a large amount of PDGF, and the amount was the largest in "thawed + frozen three times". The commercial PL was used as a mixture of PURE: HELIOS UltraGRO-PURE, PURE GMP: HELIOS UltraGRO-PURE (GMP Grade) and Advanced GMP: HELIOS UltraGRO-Advanced (GMP Grade).

[Table 1]

|  | FBS | commercial PL | ① thawed only | ② thawed + frozen once | ③ thawed + frozen twice | ④ thawed + frozen three times |
|---|---|---|---|---|---|---|
| **number of examination** | 3 | 3 | 5 | 10 | 10 | 10 |
| Average(ng/ml) | 1.86 | 9.62 | 8.93 | 15.35 | 17.89 | 18.29 |
| SE | 0.41 | 0.58 | 1.90 | 2.05 | 2.21 | 2.02 |

(A-2) PDGF

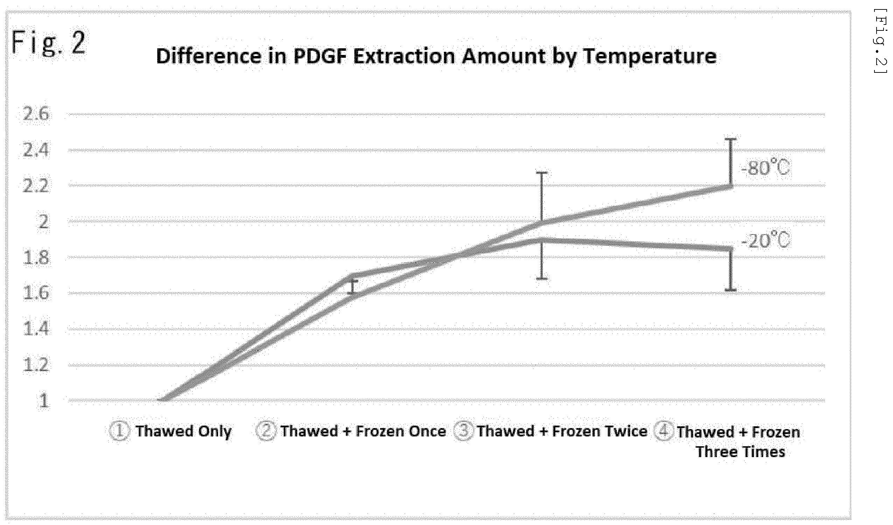

Table 2 shows a comparison of samples frozen at -20°C and at -80°C. Figure **2** is a graphical representation of Table 2. Repeated freezing and thawing at -20°C or -80°C increased PDGF (where the amount was 2.2 times higher in the "thawed + frozen three times" frozen at -80°C compared to the "thawed only"), and more PDGF was extracted at -80°C than -20°C in the third freezing.

[Table 2]

| | | ① thawed only | ② thawed + frozen once | ③ thawed + frozen twice | ④ thawed + frozen three times |
|---|---|---|---|---|---|
| ave | -20°C | 1 | 1.7 | 1.9 | 1.85 |
| | -80°C | 1 | 1.58 | 1.99 | 2.2 |
| se | -20°C | 0 | 0.09 | 0.28 | 0.26 |
| | -80°C | 0 | 0.1 | 0.22 | 0.23 |

(B-1) Brain-Derived Neurotrophic Factor (BDNF)

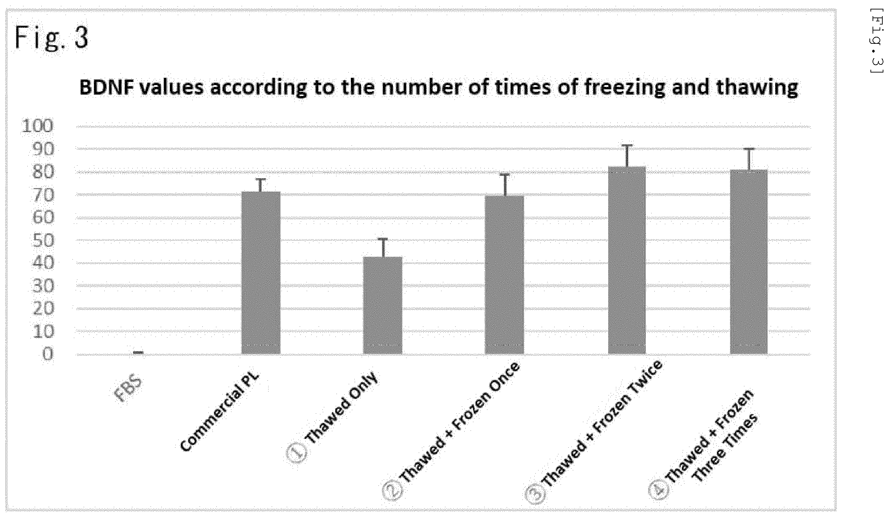

Table 3 summarizes the amount of brain-derived neurotrophic factor (BDNF) in each sample. Figure **3** is a graphical representation of Table 3. Similar to PDGF, repeated freezing and thawing increased BDNF in solution (Table 3). FBS contained almost no BDNF. The amount of BDNF was higher in the commercial product than in the "thawed only", and the amount was even higher in the samples that were frozen and thawed one to three times. The amount was higher in the "thawed + frozen twice" and "thawed + frozen three times" than in the "thawed + frozen once", and the amounts of BDNF in "thawed + frozen twice" and "thawed + frozen three times" were similar.

[Table 3]

| | FBS | commercial PL | ① thawed only | ② thawed + frozen once | ③ thawed + frozen twice | ④ thawed + frozen three times |
|---|---|---|---|---|---|---|
| n | 3 | 3 | 5 | 10 | 10 | 1 0 |
| Average | 0.54 | 71.50 | 42.58 | 6.70 | 82.19 | 80.61 |
| SE | 0.25 | 5.25 | 7.87 | 9.12 | 9.47 | 9.46 |

(B-2) BDNF

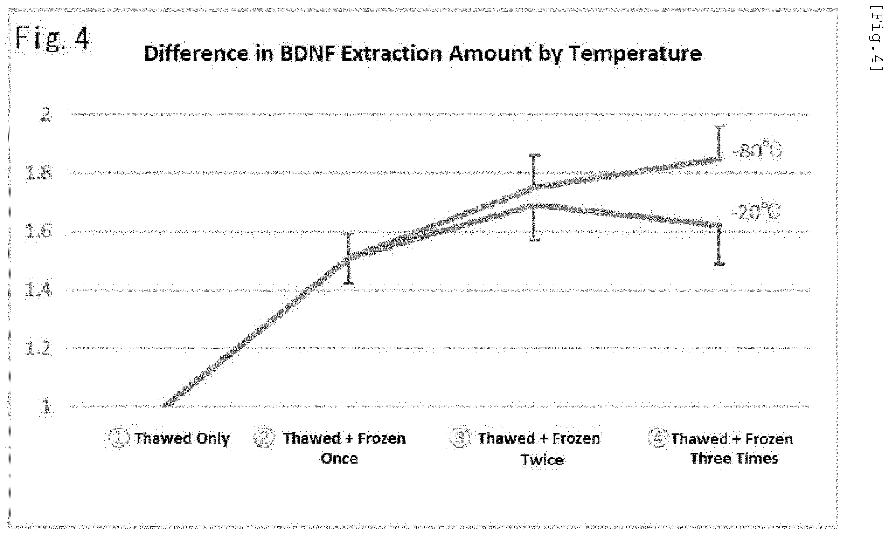

Table 4 shows a comparison of samples frozen at -20°C and - 80°C. Figure **4** is a graphical representation of Table 4. Repeated freezing and thawing at -20°C or -80°C increased BDNF (where the amount was 1.8 times higher in the "thawed + frozen three times" frozen at -80°C compared to the "thawed only"). In the "thawed + frozen three times", more BDNF was extracted when frozen at -80°C than when frozen at -20°C.

[Table 4]

| | | ① thawed only | ② thawed + frozen once | ③ thawed + frozen twice | ④ thawed + frozen three times |
|---|---|---|---|---|---|
| ave | -20°C | 1 | 1.51 | 1.69 | 1.62 |
| | -80°C | 1 | 1.51 | 1.75 | 1.85 |
| se | -20°C | 0 | 0.08 | 0.11 | 0.11 |
| | -80°C | 0 | 0.09 | 0.12 | 0.13 |

(C-1) Transforming growth factor $\beta1$ (TGF$\beta1$)

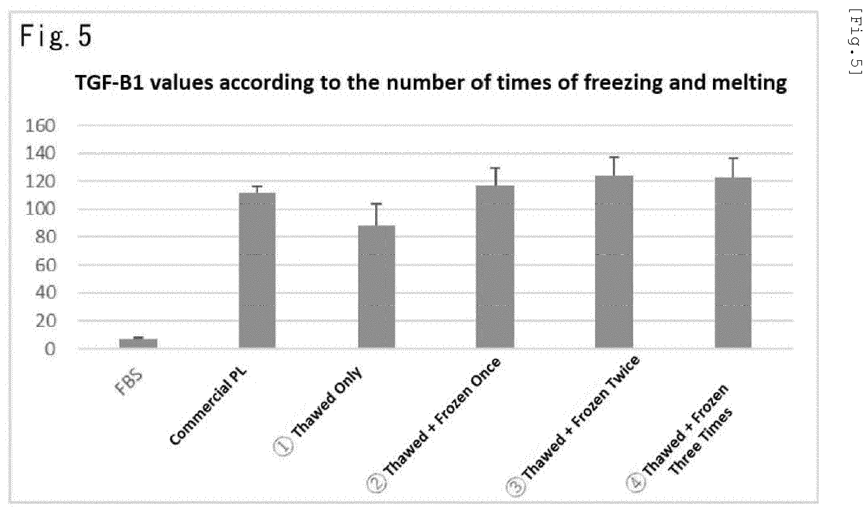

Table 5 summarizes the amount of transforming growth factor $\beta1$ (TGF$\beta1$) in each sample. Figure **5** is a graphical representation of Table 5. Repeated freezing and thawing increased TGF$\beta1$ in solution. FBS contained almost no TGF$\beta1$. The amount of TGF$\beta1$ was higher in the commercial product than in the "thawed only", and the amount was even higher in the samples that were frozen and thawed one to three times. The amount was higher in the "thawed + frozen twice" and "thawed + frozen three times" than in the "thawed + frozen once", and the amounts of TGFβ1 in "thawed + frozen twice" and "thawed + frozen three times" were similar.

[Table 5]

|  | FBS | commercial PL | ① thawed only | ② thawed + frozen once | ③ thawed + frozen twice | ④ thawed + frozen three times |
|---|---|---|---|---|---|---|
| n | 3 | 3 | 5 | 10 | 10 | 10 |
| Average | 7.36 | 111.47 | 87.94 | 116.82 | 12:3.99 | 122.47 |
| SE | 0.68 | 4.50 | 16.24 | 12.91 | 13.24 | 14.31 |

(C-2) TGFβ1

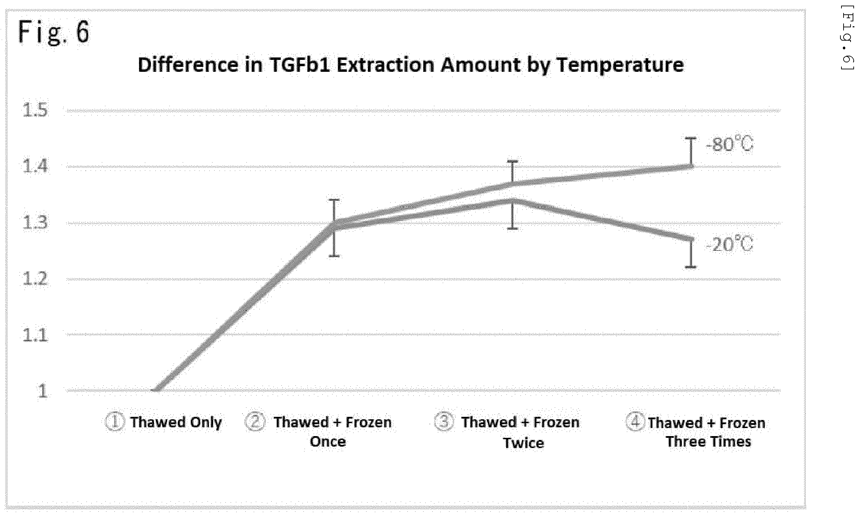

Table 6 shows a comparison of samples frozen at -20°C and - 80°C. Figure **6** is a graphical representation of Table 6. Repeated freezing and thawing at -20°C or -80°C increased TGFβ1 (where the amount was 1.4 times higher in the "thawed + frozen three times" frozen at -80°C compared to the "thawed only"). In the "thawed + frozen three times", more TGFβ1 was extracted when frozen at -80°C than when frozen at -20°C.

[Table 6]

|  |  | ① thawed only | ② thawed + frozen once | ③ thawed + frozen twice | ④ thawed + frozen three times |
|---|---|---|---|---|---|
| ave | -20°C | 1 | 1.29 | 1.34 | 1.27 |
|  | -80°C | 1 | 1.3 | 1.37 | 1.4 |
| se | -20°C | 0 | 0.04 | 0.04 | 0.05 |
|  | -80°C | 0 | 0.05 | 0.05 | 0.05 |

(Discussion)

From the above results, it was found that repetition of freezing and thawing multiple times, and three times in particular, increases trophic factors in the platelet lysate, which suggests that such platelet lysates can be advantageously used in cell culture. In addition, setting the freezing temperature to -80°C resulted in the secretion of many trophic factors. Freezing at -80°C is advantageous because it shortens the freezing time and also suggests efficient cell culturing. Therefore, freezing and thawing are preferably performed twice or more, and more preferably at a temperature lower than -20°C (such as -80°C).

(Example 2: Differences in Trophic Factor Extraction with or without Inactivation after Freezing and Thawing)

(Materials and Methods)

A concentrated platelet solution (made by JRCS) collected in a liquid state was placed in freezing bags (Nipro Froze Bag F-100; Catalog No. 89-100, 50 ml) and stored at -80°C. The concentrated platelet solution was then thawed overnight in a refrigerator at 4°C ("thawed only"). After freezing and thawing by various methods, 0.25 ml of heparin was mixed with the obtained platelet lysate (10 ml) and centrifuged multiple times. The platelet lysate created thereafter was divided into the platelet lysate to be inactivated (at 58°C for 1 hour) and the platelet lysate not to be inactivated ("without inactivation"). The platelet lysate was then mixed with 100 ml of MEMα and passed through a filter (0.45 um) (the filter to be changed if clogged). ELISA was then performed.

(Results)

By not performing inactivation, PDGF increased by 20% and BDNF by 10%, but TGFβ1 remained unchanged. By not performing inactivation, the number of processes for changing a clogged filter with a new one was greatly reduced (about 1/10).

(A) PDGF

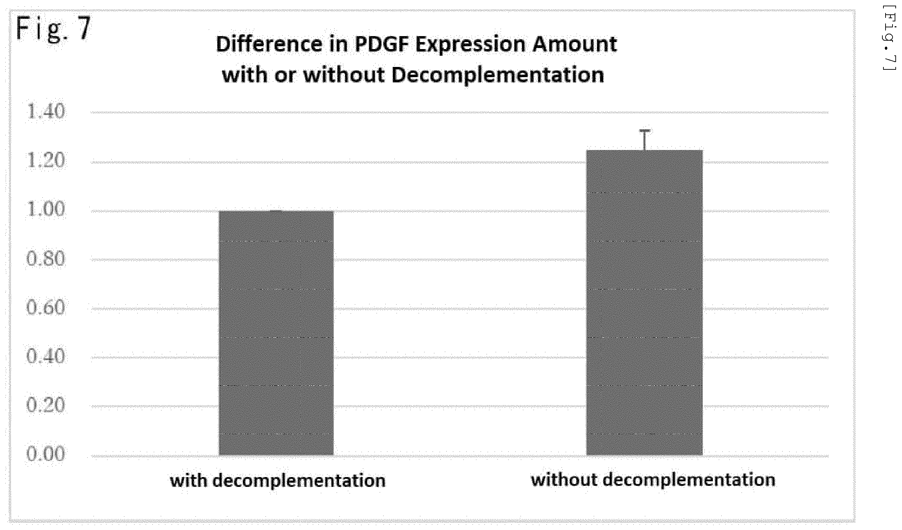

Table 7 summarizes the amount of PDGF in the inactivated and non-inactivated samples. Figure 7 is a graphical representation of Table 7.

[Table 7]

|  | with decomplementation | without decomplementation |
| --- | --- | --- |
| ave | 1.00 | 1.25 |
| sd | 0 | 0.48 |
| se | 0 | 0.08 |

(B) BDNF

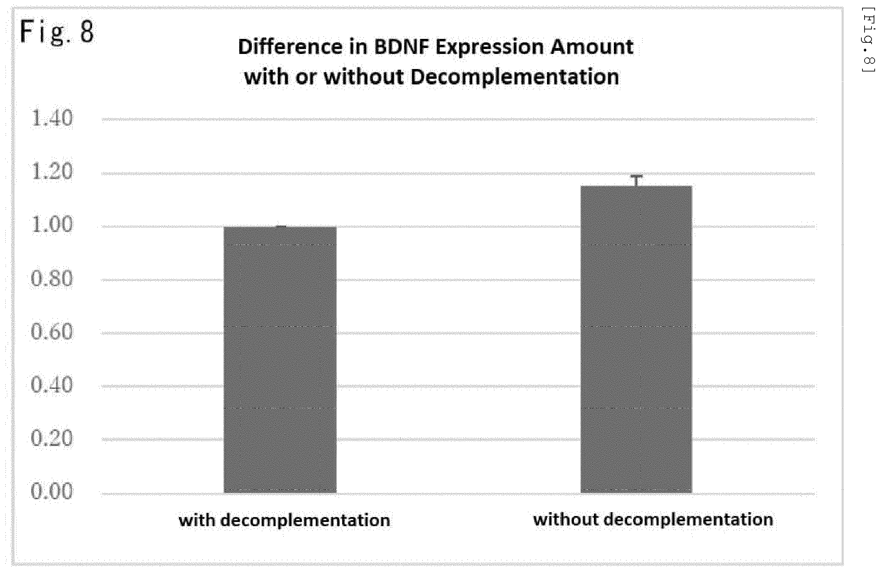

Table 8 summarizes the amount of BDNF in the inactivated and non-inactivated samples. Figure **8** is a graphical representation of Table 8.

[Table 8]

|  | with decomplementation | without decomplementation |
| --- | --- | --- |
| ave | 1.00 | 1.15 |
| sd | 0 | 0.26 |
| se | 0 | 0.04 |

(C) TGF$\beta$1

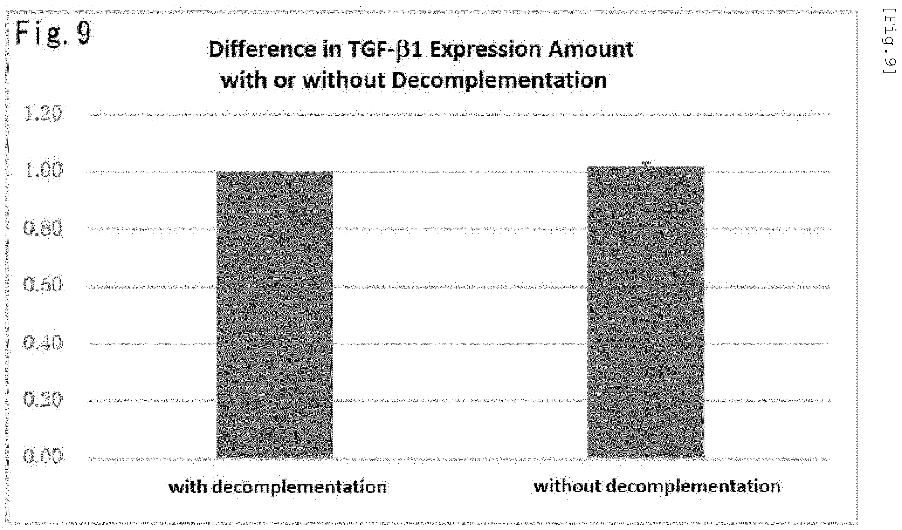

Table 9 summarizes the amount of TGF$\beta$1 in the inactivated and non-inactivated samples. Figure **9** is a graphical representation of Table 9.

[Table 9]

|  | with decomplementation | without decomplementation |
| --- | --- | --- |
| ave | 1.00 | 1.02 |
| sd | 0 | 0.07 |
| se | 0 | 0.01 |

(Example 3: Differences in Cell Culture Rate)

(Materials and Methods)

As shown in Table 10, bone marrow stem cells (n=3) were cultured by four methods (inactivation: yes and freezing and thawing: no, inactivation: no and freezing and thawing: no, inactivation: yes and freezing and thawing: yes, inactivation: no and freezing and thawing: yes), with and without inactivation and with and without freezing and thawing twice (freezing and thawing a total of 3 times because bone marrow stem cells that have already been frozen are used).

[Table 10]

|  | with decomplementation | without dacomplementation (non-decomplementation) |
| --- | --- | --- |
| No freezing or thawing (melting only) | Name A | Name B |
| Freezing and thawing twice additionally (3 times in total) | Name C | Name D |

Fresh bone marrow fluid was collected, the number of cells was counted in the three steps below, and with the A used as a reference, the number of times the number of cells increased was calculated.

Step 1: Seed $1 \times 10^7$ mononuclear cells in a 25cm$^2$ flask, culture them, and count the number after 8 days
Step 2: Harvest the cells from Step 1, re-seed 45000 out of the total cells, and count the number after 1 week
Step 3: Harvest the cells from Step 2, re-seed 45000 out of the total cells, and count the number after 1 week

(Results)

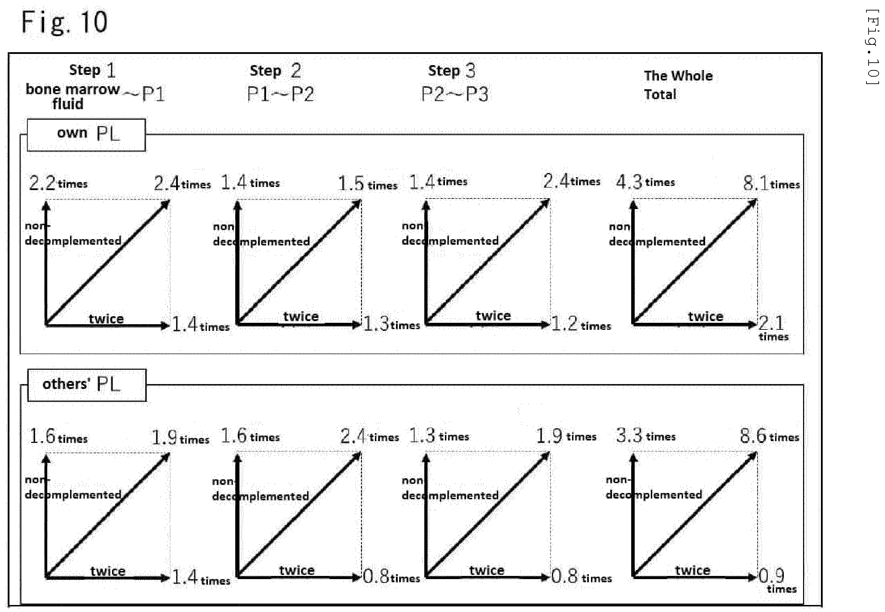

By performing freezing and thawing three times and not performing inactivation, the number of cells that can be produced in the same amount of time increased by about eight times (Figure 10). Further, interestingly, both freezing and thawing three times and non-inactivation increased trophic factors, and unexpectedly, cell proliferation was accelerated synergistically. There was no difference in the culture rate between one's own PL (PL obtained from a subject whose bone marrow fluid was collected, i.e., autologous PL) and others' PL (allogeneic PL) due to non-inactivation.

(Example 4: Quality of Stem Cells)

(Trophic Factor Secretion)

(Materials and Methods)

Trophic factors (bNGF, HGF) in the culture supernatant were detected by ELISA. A culture supernatant of P3 was used as a sample and cultured by the method below. Since the culture supernatant was used, the concentration per cell was calculated by dividing by the number of cells, and the results were compared.
Allogeneic Conventional Method: Human BMSCs (bone marrow-derived mesenchymal stem cells) were cultured using allogeneic platelet lysate that had been thawed only once, according to Example 1.
New Autologous -80°C Method: Human BMSCs were cultured using autologous platelet lysate that had been frozen and thawed three times at -80° and not inactivated, according to Example 3.

(Results)

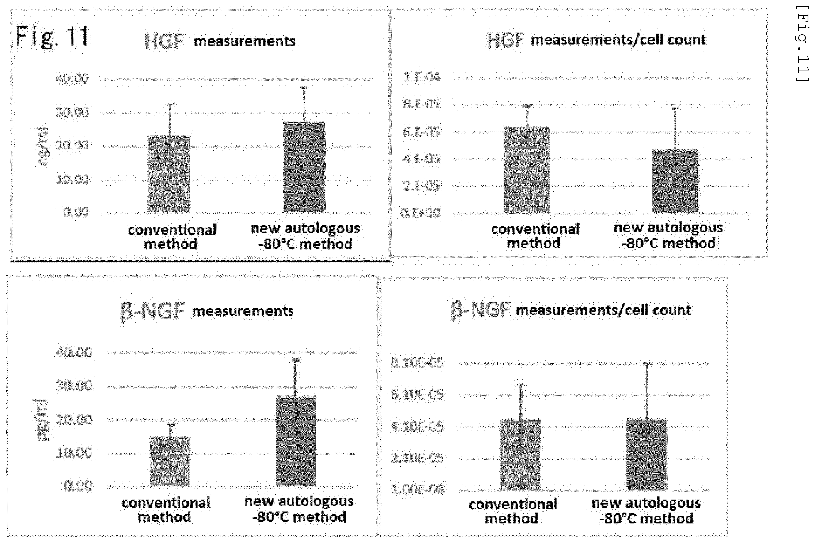

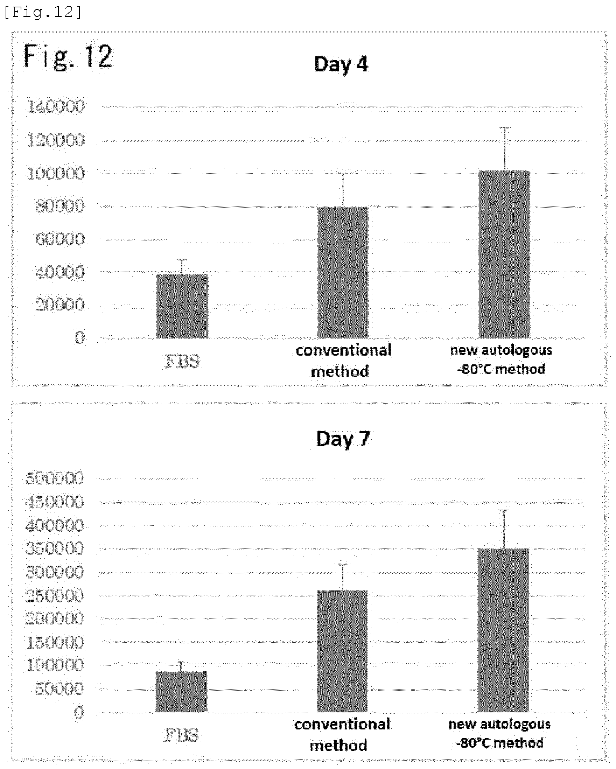

The results are shown in Figure 11. While the total amount of HGF in the culture solution increased with the new autologous -80°C method, the result was slightly less when divided by the number of cells (no significant difference) because the number of cells in the new autologous -80°C method was increased. In addition, bNGF was secreted at the same level per cell. Therefore, in summary, although the culture rate increased, the amount of trophic factors secreted by the produced cells did not change (to the same extent); it was not that poor quality cells were produced.
Furthermore, Figure 12 shows the results of the number of cells after 4 days and 7 days after culturing at 1500 cells/cm$^2$. As shown, the PL produced by the new autologous -80°C method showed a marked increase in cell number compared to the conventional method (Figure 12).

(Gene Expression)

(Materials and Methods)

Human BMSCs (n = 4) created with allogeneic PL that had been thawed only once (hereinafter referred to as conventional method) and human BMSCs (n = 3) that were created with autologous PL that had been frozen and thawed three times and non-inactivated (hereinafter referred to as new autologous -80°C method) were analyzed by microarray (Affymetrix, GeneChip WT PLUS Reagent Kit). Based on the results, the genes whose expression was enhanced ($p < 0.05$ or 0.01 and Log ratio > 1 or 3 (expression ratio 2 or 8 times or more)), or the genes whose expression was decreased ($p < 0.05$ or 0.01 and Log ratio < -1 or -3 (expression ratio 1/2 or 1/8 times or less)), by the new autologous -80°C method were extracted using Transcriptome Viewer (Kurabo), with the conventional method used as a control.

(Results)

Strictly judging, out of 20,000 genes, the expression of one hundred genes was clearly enhanced, while the expression of another one hundred genes was clearly decreased. When their relationships were examined by GO term, some of the cells with enhanced expression were associated with cell division (2, 3 and 7) and some with tissue repair (6). On the other hand, the cells with decreased expression were factors for the promotion of cell differentiation (6, 7, 18, 19, 24, 32, 37, 41 , that is, undifferentiation is kept) and for promoting cell death (32, that is, increase in survival rate).

[Table 11]

| Genes with Enhancement | | |
|---|---|---|
| **All** 21446 **Genes** | Log ratio >1 | Log ratio >3 |
| p<0.05 | 1306 **Genes** (A) | 86 **Genes** (B) |
| p<0.01 | 832 **Genes** (C) | 71 **Genes** (D) |
| **Genes with Decrease** | | |
| **All** 24351 **Genes** | Log ratio <-1 | Log ratio <-3 |
| p<0.05 | 1162 **Genes** (E) | 107 **Genes** (F) |
| p<0.01 | 730 **Genes** (G) | 71 **Genes** (H) |

Analysis of the D, which showed the most severe enhancement among the genes, by Panther GO analysis revealed that the expression of genes related to the following was enhanced:

1. Protein localization to cytoskeleton
2. Regulation of mitotic metaphase/anaphase transition
3. Cell cycle checkpoint
4. Chromosome segregation
5. Extracellular matrix organization
6. Wound healing
7. Cell division
8. Mitotic Cell Cycle process
9. Tissue development
10. Animal organ development

Further, similarly, analysis of the most severe H by Panther GO analysis revealed that the expression of genes related to the following was decreased:

1. Histone dephosphorylation
2. Very-low-density lipoprotein particle clearance
3. regulation of cardioblast proliferation
4. Chylomicron remnant clearance
5. pharyngeal system development
6. artery morphogenesis
7. regulation of bone mineralization
8. Response to cAMP
9. negative regulation of small molecule metabolic process
10. Regulation of receptor signaling pathway via JAK-STAT
11. Negative regulation of neuron apoptotic process
12. Extracellular matrix organization
13. Positive regulation of peptidyl-tyrosine phosphorylation
14. Response to reactive oxygen species
15. Positive regulation of stress-activated MAPK cascade
16. Positive regulation of protein kinase B signaling
17. Regulation of JNK cascade
18. Striated muscle tissue development
19. Regulation of epithelial cell proliferation
20. Regulation of ERK1 and ERK2 cascade
21. Regulation of MAP kinase activity
22. Positive regulation of protein serine/threonine kinase activity
23. Response to acid chemical
24. Muscle structure development
25. Cytokine-mediated signaling pathway

26. Cellular response to hormone stimulus
27. Response to lipid
28. Cellular response to growth factor stimulus
29. Enzyme linked receptor protein signaling pathway
30. Regulation of cell adhesion
31. Positive regulation of cell differentiation
32. Positive regulation of cell death
33. Regulation of cell migration
32. Anatomical structure formation involved in morphogenesis
33. Positive regulation of cell population proliferation
34. Cellular response to oxygen-containing compound
35. Animal organ morphogenesis
36. Cell migration
37. Positive regulation of multicellular organismal process
38. Regulation of cell cycle
39. Positive regulation of transcription, DNA-templated
40. Regulation of immune system process
41. Cell differentiation

(Example 5: Cells of other species (rat) do not grow in PL produced by non-inactivation)

(Materials and Methods)

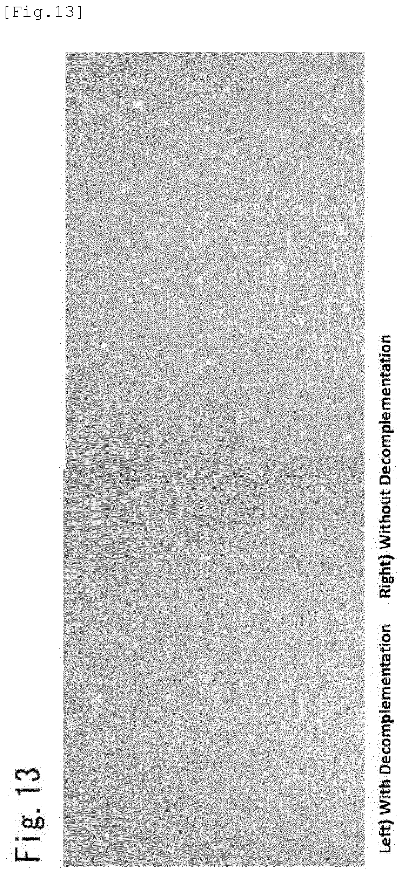

Rat BMSCs were harvested and P2-4 were used for culture. The culture media used were those inactivated and non-inactivated with human PL.
Cells engrafted and divided normally in the inactivated medium, while no cells survived at all in the non-inactivated medium (Figure **13**). It is believed that the rat cells were unable to grow because the unknown components, which are supposed to be removed as a result of the inactivation, were not removed. As shown in Example 3, the cells were able to grow with non-inactivated human PL, even if it is someone else's PL, in the same way as with the inactivated PL. In consideration of the results, however, it means that there is a risk of immune extermination even when human stem cells are grown with other people's PL; thus, the use of autologous PL is considered to be highly safe.

(Example 6: Temperature for freezing and thawing)

(Materials and Methods)

Experiment (1)

Changes in growth factors by platelet freezing temperature (-10, -20, -30, -80, -196°C) and freezing time (0, 90, 120, 180, 240 min)

Experiment (2)

Changes in growth factors in -80°C frozen platelets by thawing temperature (4°C (overnight), 20°C (overnight), 37°C (1 hour))

Experiment (3)

Confirmation of growth factors at -80°C and -196°C (liquid nitrogen) freezing, and confirmation of culture proliferation of H-BMSC using the PL
In all experiments, transfer portion of JRCS's platelet concentrate formulation was used. PDGF-BB Total-BDNF TGF-$\beta$1 was measured using an ELISA Kit (R&D Inc.).
In Experiments (1) and (2), the PL was produced by the new -80°C method. In Experiment (3), the PL was produced by a clinical trial method and the new -80°C method for -80°C freezing, while the PL was produced by a new -180°C method for -196°C freezing.
The culture solution used was MEM-$\alpha$ + penicillin-streptomycin mixture + 10% of each PL.

Explanation of Terms

Clinical Trial Method (Conventional Method): those frozen at -80°C are thawed and used as they are. Inactivation is performed (60°C for 30 minutes). After that, sterilization is performed using a filter, but the filter needs to be changed (about 10 times) because it clogs quickly.

New -80°C Method: those frozen at -80°C are thawed and then, or fresh platelets are, repeatedly frozen at -80°C and thawed (at various temperatures) any number of times (one to six times) . Inactivation (60°C for 30 minutes) is not performed. Sterilization is performed using a filter; however, the method caused almost no clogging in the filter.

New -180°C Method: those frozen at -80°C are thawed and then repeatedly frozen at -180°C and thawed (at various temperatures) any number of times (one to six times). Inactivation (60°C for 30 minutes) is not performed. Sterilization is performed using a filter.

(Results)

Experiment (1)

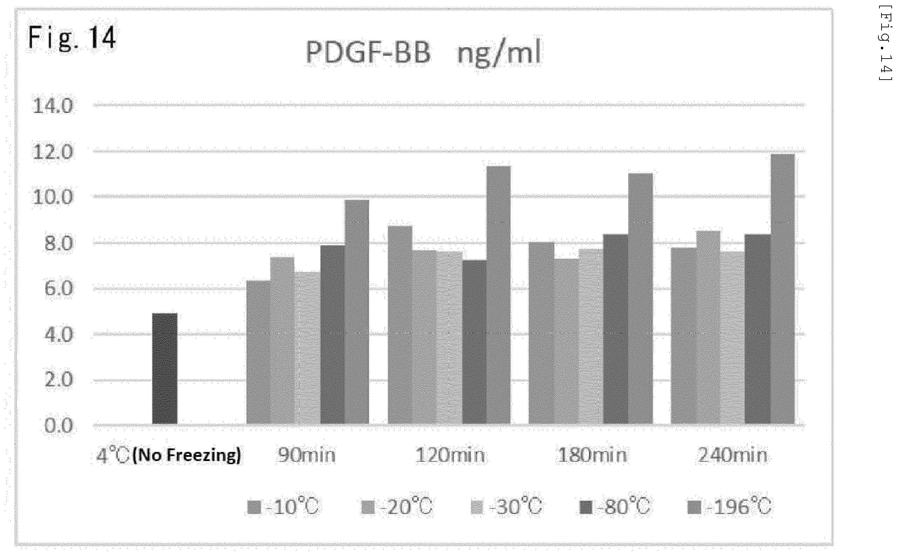

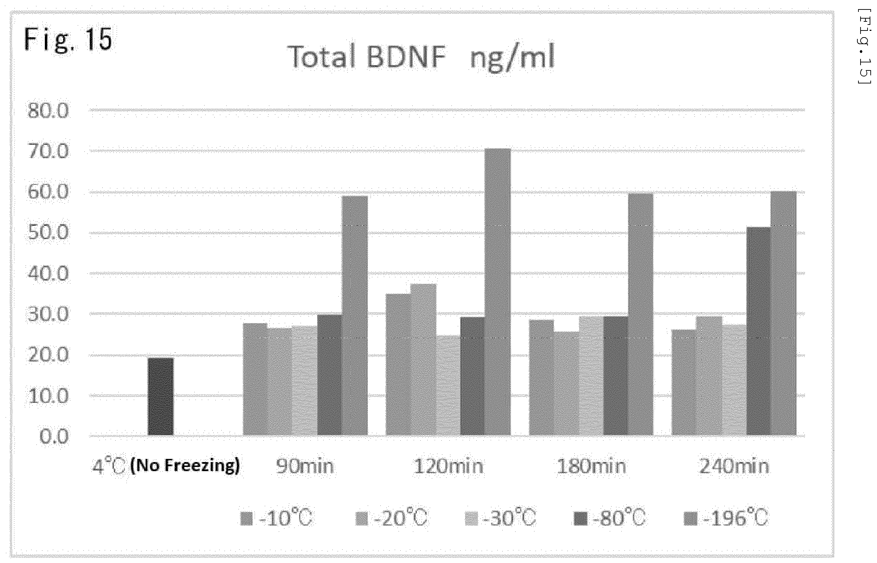

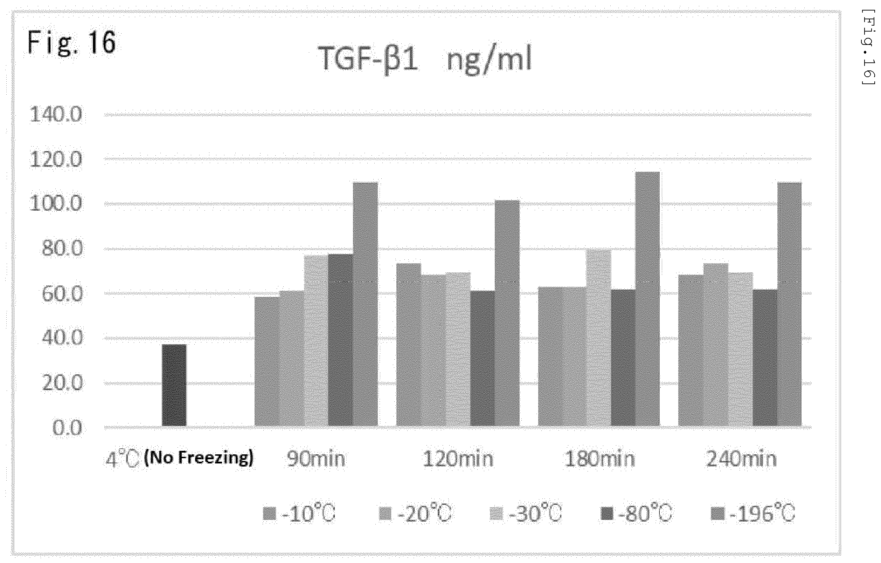

The results are shown in Figures **14** to **16.** In all the cases, significant increase was observed with the freezing in liquid nitrogen. It seemed that no particular difference was observed with regard to the freezing time.

Experiment (2)

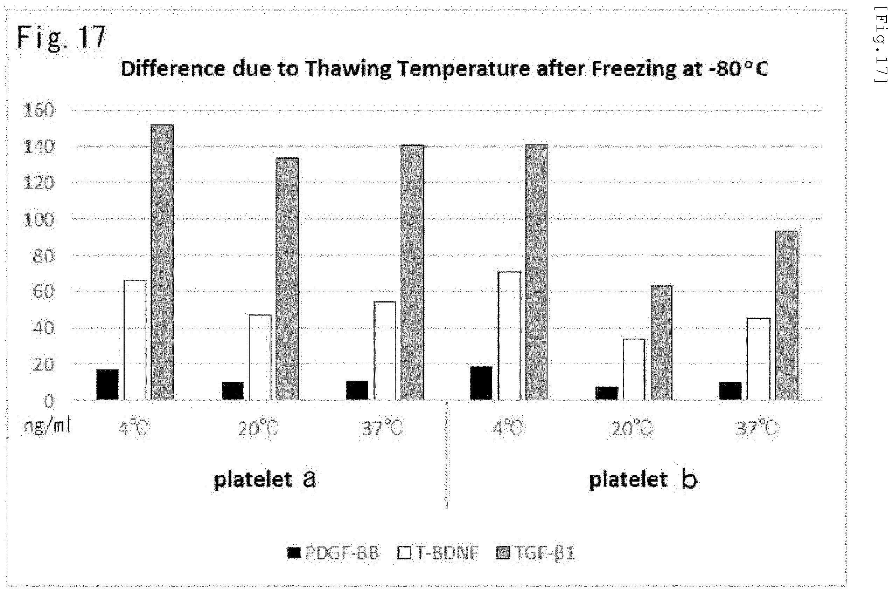

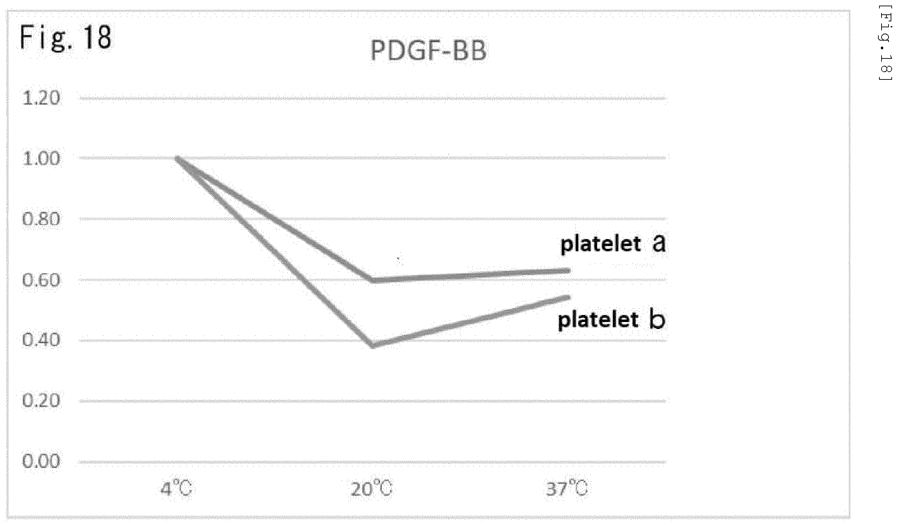

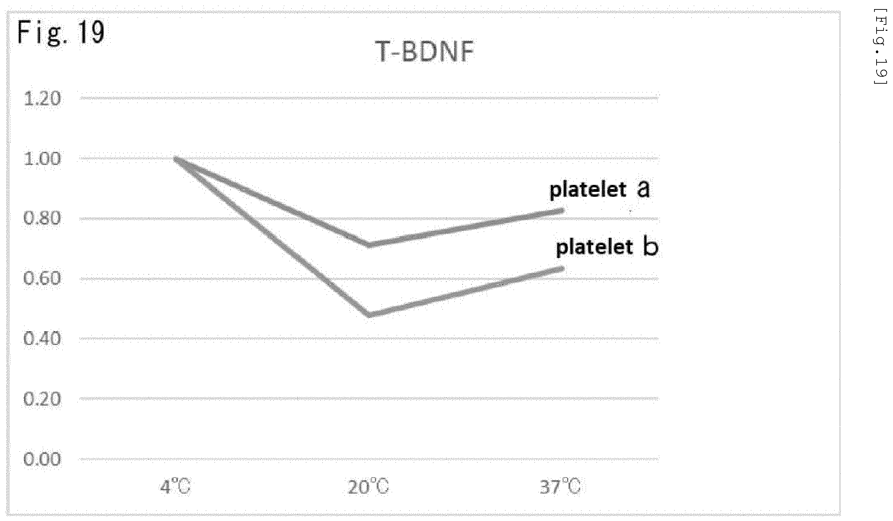

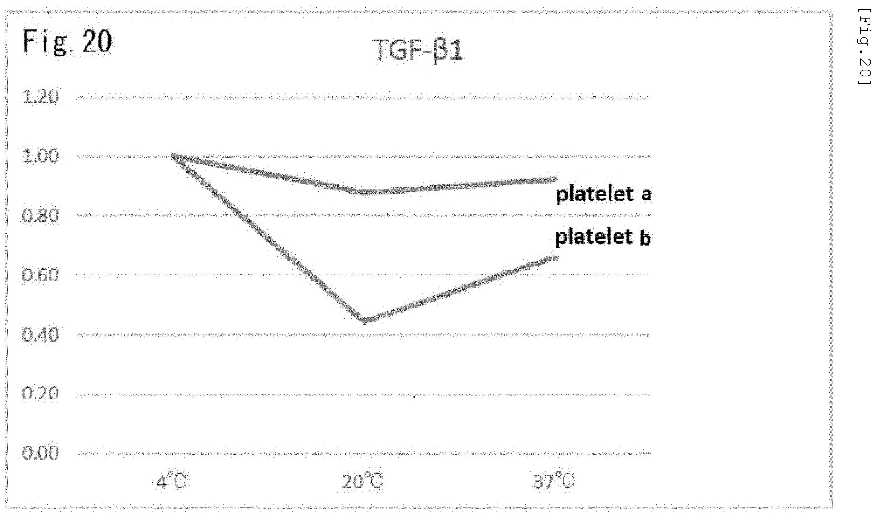

The results are shown in Figures **17** to **20.** Thawing at 4°C resulted in the greatest release of growth factors. Figures **17** to **20** show the results for two platelets (platelet **a** and platelet **b).**

Experiment (3)

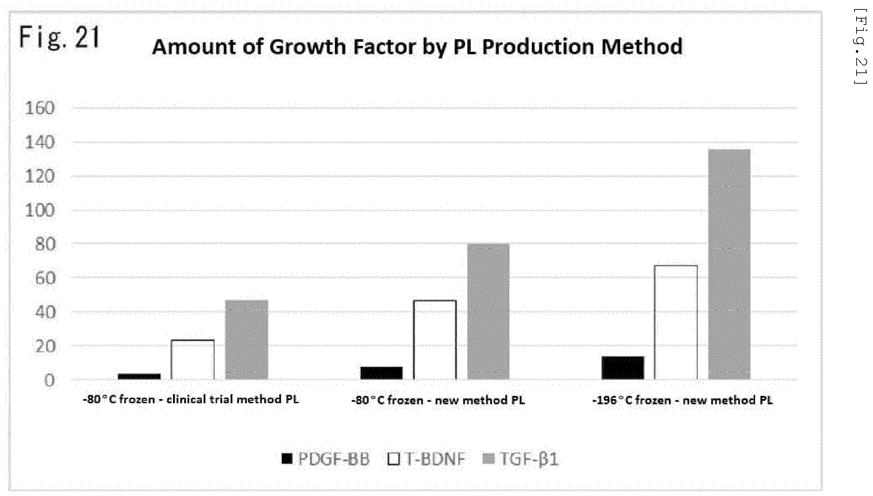

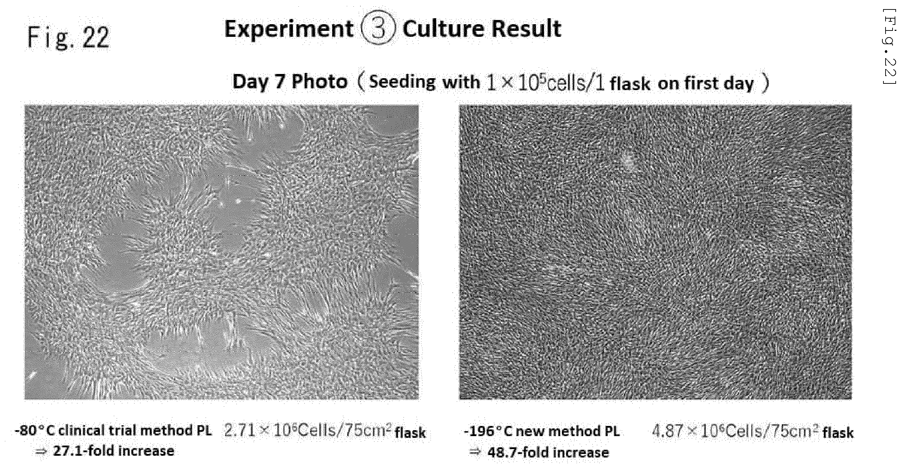

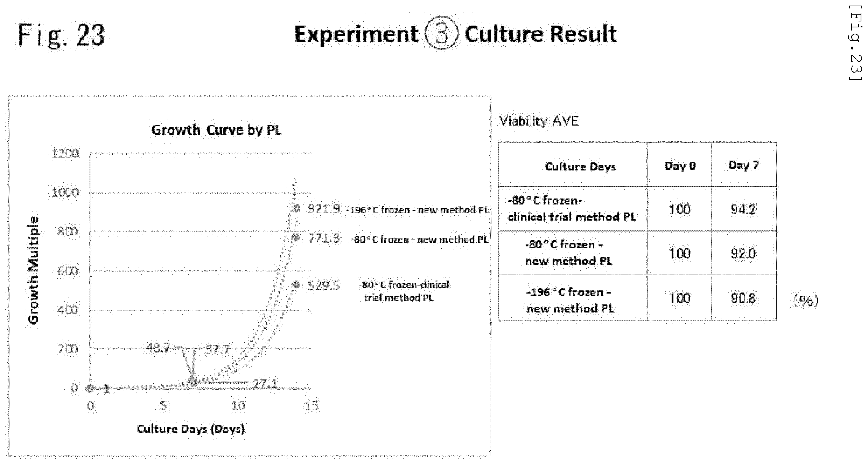

The results are shown in Figures **21** to **23.**

(Example 7: Number of Times of Freezing and Thawing)

In this example, the optimal number of times of freezing and thawing was examined. This example was carried out according to the method of Example 1. The number of times of freezing and thawing was set to one to six times.

(Results)

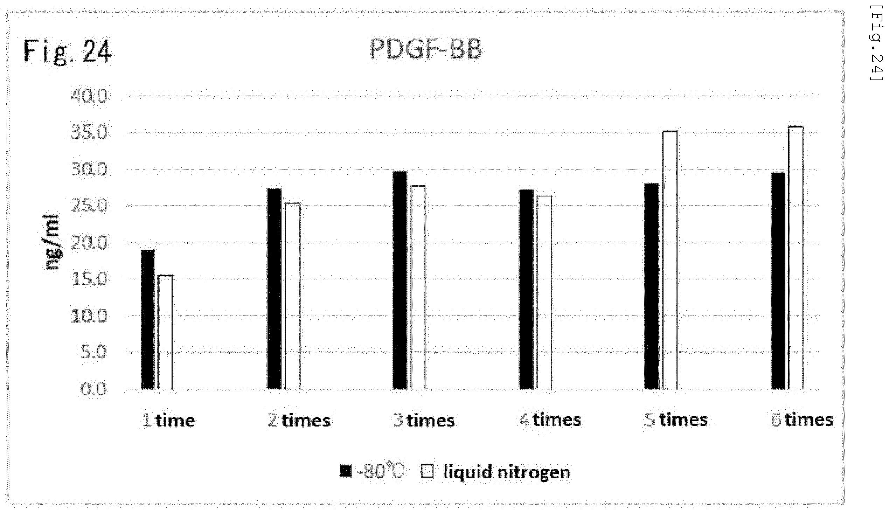

At the -80°C, almost no increase in trophic factors was observed after the third freezing and thawing. At the -196°C freezing and thawing, a slight increase was observed after the fifth time. It is possible to say that the increase almost reached a plateau by the third time for both the -80°C freezing and thawing and the -196°C freezing and thawing. In view of the above, it was suggested that the minimum number of times of freezing and thawing for efficient extraction of trophic factors is three times (Figure **24).**

(Example 7: Confirmation of Differentiation Potential)

In this example, it was determined whether stem cells cultured with the platelet lysate of the present disclosure have normal differentiation potential. Mesenchymal stem cells (MSCs) cultured using platelet lysates prepared by repeating freezing and thawing three times and without inactivation were used.

(Differentiation into Adipocytes)

(Materials)

- 12-well plate (CORNING)
- Adipocyte differentiation medium (Gibco, StemPro™ Adipogenesis Differentiation Kit)
- Normal medium (MEM-$\alpha$ + 5%PL + PS)
- Oil Red O (Cayman): stored at 4°C (at the time of use, the reagent was diluted with distilled water at a ratio of 6:4 and filtered through a 0.45 um filter)

- Four % paraformaldehyde phosphate buffer: stored at 4°C

(Methods)

(1) Seeding of BMSCs
Cells were seeded in 12-well plates at a concentration of 10000/cm$^2$ using 1 ml of normal medium. Seeded were 10000 × 4 cm$^2$ per well (well base area) = 40000 cells.
(2) Culturing of BMSCs
Cells were cultured until 80% confluent (about 2 to 3 days).
(3) The medium was removed, and 900 μL of adipocyte differentiation induction medium was added. For non-differentiated controls, 900 μL of normal medium was added.
(4) The medium was changed every 3 to 4 days (total volume).
(5) The cells were cultured for 14 days from the initiation of differentiation.
(6) Oil Red O staining was started, and the medium was removed.
(7) 1.6 ml of PBS was added, followed by washing.
(8) 0.8 ml of 4% paraformaldehyde phosphate buffer was added and fixed at room temperature for 5 minutes.
(9) The fixative was removed.
(10) 1.6 ml of PBS was added, followed by washing twice.
(11) 0.8 ml of Oil Red O staining solution was added and stained with shaking at room temperature for 15 minutes.
(12) The staining solution was removed.
(13) Washing was performed 3 times with 1.6 ml of PBS (where the washing time and the number of times of washing are appropriately changed in accordance with the intensity of staining).
(14) 0.8 ml of PBS was added, and observation was carried out under a microscope.

(Differentiation into Osteocytes)

(Materials)

- 12-well plate coated with collagen (CORNING)
- Osteocyte differentiation induction medium (Gibco, StemPro™ Osteogenesis Differentiation Kit)
- Normal medium (MEM-α + 5%PL + PS)
- Alizarin Red S (Sigma): stored at room temperature A 1% solution was prepared and stored.

    (i) One g was dissolved in 95 ml of MilliQ while stirring with a stirrer.
    (ii) The pH was adjusted to 4.2 with 0.1N HCl.
    (iii) The solution was diluted in a measuring flask to 100 ml and filtered through a 0.8 um filter.

- Four % paraformaldehyde phosphate buffer: stored at 4°C

(Methods)

(1) Seeding of BMSCs
Cells were seeded in collagen-coated 12-well plates at a concentration of 10000/cm$^2$ using 1 ml of normal medium. Seeded were 10000 × 4 cm$^2$ per well (well base area) = 40000 cells.
(2) Culturing of BMSCs (for 1 day)
(3) The medium was removed, and 800 μL of adipocyte differentiation induction medium was added. For non-differentiated controls, 800 μL of normal medium was added.
(4) The medium was changed every 3 to 4 days.
Only at the time of the first medium change, the entire amount was changed. For the second and subsequent changes, the old medium was removed with a pipette leaving about 200 μL, and 800 μL of new medium was slowly added along the wall (care must be taken as the cells are easily detached).
(5) Cells were cultured for 21 to 28 days from the initiation of differentiation.

(6) Staining with Alizarin Red was started and the medium was removed.
(7) 1.6 ml of PBS was added and washed.
(8) 0.8 ml of 4% paraformaldehyde phosphate buffer was added and fixed at room temperature for 5 minutes.
(9) The fixative was removed.
(10) 1.6 ml of PBS was added, followed by washing twice.
(11) 0.8 ml of Alizarin Red staining solution was added and stained with shaking at room temperature for 15 minutes.
(12) The staining solution was removed.
(13) Shaking and washing with 1 ml of MilliQ for 3 minutes was performed 5 times (the staining is often strong, so the number of times may be increased in some cases).
(14) One ml of MilliQ was added and observation was carried out under a microscope.

(Differentiation into Chondrocytes)

(Materials)

- 24-well plate (CORNING)
- Chondrocyte differentiation induction medium (Gibco, StemPro™ Chondrogenesis Differentiation Kit)
- Normal medium (MEM-$\alpha$ + 5%PL + PS)
- AlucianBlue (Sigma): stored at room temperature A 1% solution was prepared and stored.

    (i) Using a 50 ml tube, 0.2 g was dissolved in 20 ml of 0.1N HCl for 40 minutes with inversion mixing or shaking.
    (ii) It was filtered through a 0.8 um filter.

- Four % paraformaldehyde phosphate buffer: stored at 4°C

(1) Seeding of BMSCs
BMSCs were prepared at $1.6 \times 10^4/\mu L$, which were put in the center of respective wells, at 5 $\mu L$ = $8 \times 10^4$ per well, in a 24-well plate (micro mass culture).
(2) Culturing of BMSCs (60 minutes)
(3) The medium was removed, and 400 $\mu L$ of adipocyte differentiation induction medium was added. For non-differentiated controls, 400 $\mu L$ of normal medium was added.
(4) The medium was changed every 3 to 4 days.
The cartilage was in clumps, so care was taken not to tear it off or lose it during removal.
(5) The cells were cultured for 14 days from the initiation of differentiation.
(6) Staining with Alucian blue was started, and the medium was removed.
(7) 0.4 ml of PBS was added, followed by washing three times.
(8) 0.4 ml of 4% paraformaldehyde phosphate buffer was added and fixed at room temperature for 30 minutes.
(9) The fixative was removed.
(10) 0.4 ml of MilliQ was added, and washing and removing were then performed.
(11) 0.4 ml of Alucian blue staining solution was added and stained with shaking at room temperature for 30 minutes.
(12) The staining solution was removed.
(13) Washing was performed 5 times with 1 ml of MilliQ.
(14) 1 ml of MilliQ was added, and observation was carried out under a microscope.

(Results)

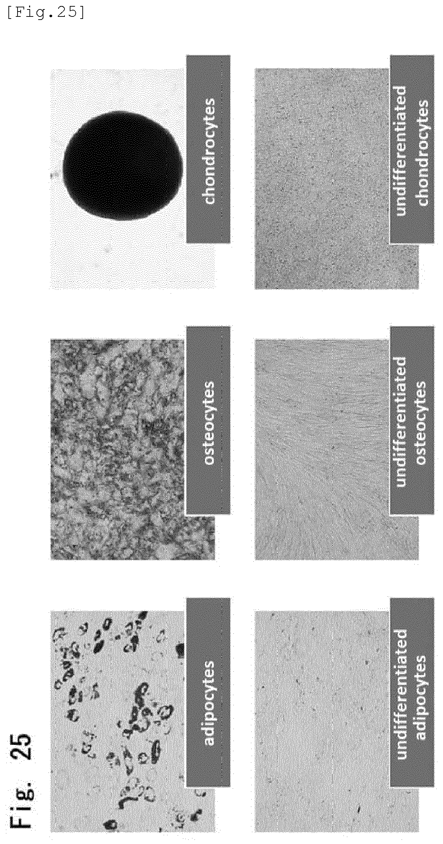

The results are shown in Figure **25.** Stem cells cultured with platelet lysates of the present disclosure were shown to have the ability to differentiate into adipocytes, osteocytes, and chondrocytes, and thus shown to have normal differentiation potential.

(Example 8: Confirmation of Exosome Secretion Ability)

In this example, the ability of the stem cells cultured with platelet lysates of the present disclosure to secrete exosomes was confirmed.

EP 4 190 892 A1

- A medium (5% PL + MEM-α) with a platelet lysate prepared by the clinical trial method (conventional method) (refer to Example 6)
- A medium (5% PL + MEM-α) with a platelet lysate prepared by the new method (refer to Example 6), in which the number of times of freezing was 3 times

Using these two types of media, hBMSCs were cultured, and changes in exosomes in the culture supernatant thereof were observed.

(Methods)

(Collection of Culture Supernatant)

(1) hBMSCs were prepared:
Cells were cultured in a 6-well plate (1500/cm$^2$, since the base area is 9 cm$^2$, 13500 cells per well are required). The cells for 3 wells were cultured per medium (Triplicate). The cells were cultured in a 2 ml medium per well. The medium was changed once every 3 days.
(2) When the cells were approximately 80 to 90% confluent, the medium was completely removed, followed by washing twice with PBS.
(3) 2 ml of PL-free basal medium (MEM-α) was added, and culturing was performed for 48 hours.
(4) The medium was collected in a falcon tube.
(5) The collected medium was filtered with a 0.2 um filter to obtain a measurement sample.
(6) The number of cells on the plate from which the supernatant was collected was counted.

(Collection of Exosome)

(1) The medium was centrifuged at 2000 G for 10 minutes to precipitate dead cells and the like.
(2) The supernatant was collected.
(3) Centrifugation was performed at 10000 G for 30 minutes at 4°C to precipitate organelles and the like.
(4) The supernatant was collected. The supernatant collected here was transferred to a tube for ultracentrifugation.
(5) Centrifugation was performed at 1,000,000 G (28500 rpm) for 70 minutes at 4°C.
(6) The precipitate was collected. The tube was turned upside down and the supernatant was completely removed with a Kimwipe. The Kimwipe was placed in the tube using tweezers and the supernatant was wiped off.
(7) The pellets were suspended in PBS or the like and subjected to a nanoparticle analyzer. Western blot was performed (Sample Buffer).

(Results)

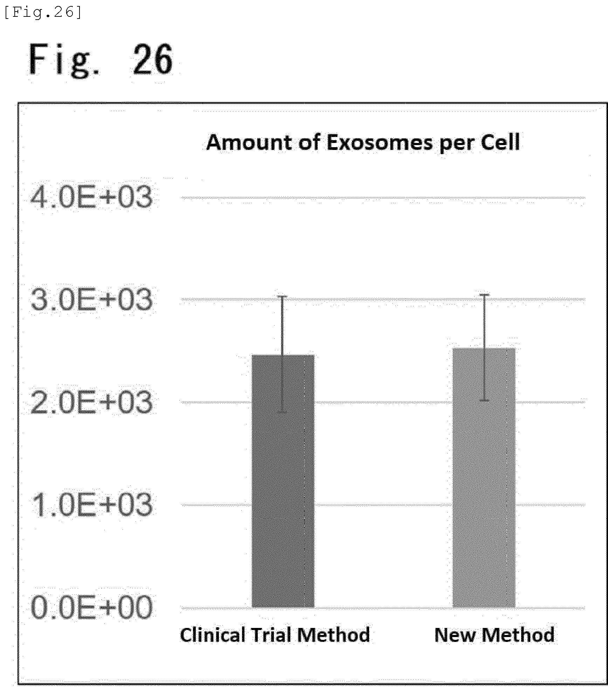

Exosomes secreted from MSCs have various organ-improving effects such as neurological function-improving effects. Thus, such exosomes were identified as an indicator of MSC quality. The results are shown in Figure **26.** The amount of exosomes secreted was not significantly different from that of the platelet lysate produced by the conventional method. Considering also the results of Example 4, which showed that the amount of trophic factor secreted was comparable between the cells cultured with the platelet lysate of the conventional method and the cells cultured with the platelet lysate of the present disclosure, the cells cultured with the platelet lysate of the present disclosure were demonstrated to be of comparable quality to the cells by the conventional method.

(Example 9: Confirmation of Cell Surface Marker)

In this example, it was confirmed whether mesenchymal stem cells cultured with the platelet lysates of the present disclosure met the criteria for cell surface markers. Mesenchymal stem cells are generally defined as fibroblast-like cells that have the ability to adhere to the bottom of plastic flasks, and the International Society for Cell Therapy indicates the condition that "cell surface markers CD105, CD73, and CD90 are positive, and CD45, CD34, CD14, CD11b, CD79α, CD19, and HLA-DR are negative." Thus, expression of these cell surface markers was confirmed.

(Materials)

- FACS Buffer (2% FBS-added PBS (-))

Six ml of FBS was added to 300 ml of PBS (-).

(Methods)

(1) Cell preparation was conducted.
Cells were prepared so that they would be $3 \times 10^5$ to $1 \times 10^6$ per tube. The cells were washed with an appropriate amount of FACS Buffer.
(2) Blocking was performed with an Fc blocker.
Every $1 \times 10^6$ cells were suspended in 100 μL of FACS Buffer, and 5 μL of Human BD FcBlock was added thereto (for $3 \times 10^5$ cells, 500 μL FACS Buffer, and 25 μL FcBlock). Incubation was performed at room temperature for 10 minutes in the dark. The cells were carried on to the next step without washing.
(3) The cells were divided into three equal parts (unstained, stain A, stain B).
(4) The cells were stained with antibodies.
Each antibody was added in the following volumes (where up to $1 \times 10^6$ can be stained with the following volumes).

|        |            |                            |
|--------|------------|----------------------------|
| Stain A: | CD79a-PE | 1 μL |
|          | CD73-BV421 | 1 μL |
|          | CD90-APC | 1 μL |
|          | CD19-APC-H7 | 1 μL |
|          | CD34-FITC | 1 μL |
|          | CD45-V500 | 1 μL |
| Stain B: | CD11b-PE | 1 μL |
|          | CD105-BV421 | 1 μL |
|          | HLA-DR-APC | 1 μL |
|          | CD14-APC-H7 | 1 μL (all are BD antibodies) |

Each was added to an aliquot of the cell suspended liquid, followed by incubating at 4°C for 30 minutes in the dark.
(5) Washing
Washing was performed twice with 2 ml of FACS Buffer (250 G, 5 minutes).
(6) Pre-Measurement Preparation
The washed cells were suspended in 500 μL of FACS Buffer. The cells were placed in a tube with a cell strainer cap (BD, 352235) (passed through a filter). The cells were stored in the dark at 4°C until measurement. Immediately before the measurement, 5 μL of 7-AAD was added (final concentration: 0.25 ug/ml).

(Results)

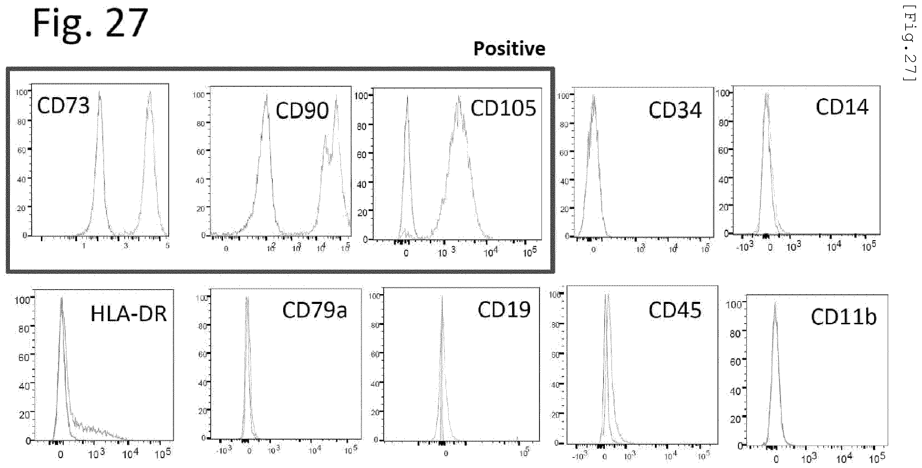

The results are shown in Figure **27**. The cells were positive for CD73, CD90 and CD105, while they were negative for D45, CD34, CD14, CD11b, CD79α, CD19 and HLA-DR, meeting the criteria for cell surface markers of mesenchymal stem cells.
From the above, the platelet lysate of the present disclosure was able to promote cell proliferation, and the cultured mesenchymal stem cells maintained normal quality.
As described above, the present disclosure is exemplified by the use of its preferred embodiments. However, the scope of the present disclosure should be interpreted solely based on the Claims. It is also understood that any patent, any patent application, and any references cited herein should be incorporated herein by reference in the same manner as the contents are specifically described herein.
The present application claims the benefit of priority to Japanese Patent Application No. 2020-130744 filed on July 31, 2020, and the entire content thereof is incorporated herein by reference.

[Industrial Applicability]

Provided is a platelet lysate (PL) used as a culture medium capable of culturing cells with high efficiency or a method of manufacturing same. The cultured cells are used for cell injection therapy and thus can be used in fields such as pharmaceuticals.

**Claims**

1.  A method for preparing a platelet lysate for use in culturing cells obtained from an individual, the method comprising the step of:
    freezing and thawing platelets obtained from the individual or equivalents thereof to obtain a platelet lysate, wherein the method does not comprise the step of inactivation.

2.  A method for culturing cells obtained from an individual, the method comprising the steps of:

    A) obtaining the cells from an individual;
    B) obtaining platelets from the individual or equivalents thereof;
    C) freezing and thawing the platelets or the equivalents thereof to obtain a platelet lysate without inactivation; and
    D) culturing the cells in the presence of the platelet lysate.

3.  A method for culturing cells obtained from an individual to obtain a cell product, the method comprising the steps of:

    A) obtaining the cells from an individual;
    B) obtaining platelets from the individual or equivalents thereof;
    C) freezing and thawing the platelets or the equivalents thereof to obtain a platelet lysate without inactivation; and
    D) culturing the cells in the presence of the platelet lysate and optionally forming the obtained cells into a cell product.

4.  The method of any one of claims 1 to 3, wherein the platelets or the equivalents thereof are frozen and thawed twice or more.

5.   The method of claim 4, wherein the platelets or the equivalents thereof are frozen and thawed three times.

6.  The method of any one of claims 1 to 5, wherein the platelets or the equivalents thereof are frozen at least once at a temperature below -20°C.

7.  The method of any one of claims 1 to 6, wherein the platelets or the equivalents thereof are frozen at least once at a temperature of -30°C or lower.

8.  The method of any one of claims 1 to 7, wherein the platelets or the equivalents thereof are frozen at least once at a temperature of -80°C or lower.

9.  The method of any one of claims 1 to 8, wherein the platelets or the equivalents thereof are frozen at least once at a temperature of -80°C to -196°C.

10. The method of any one of claims 1 to 9, wherein the platelets or the equivalents thereof are frozen at least once at a temperature of -80°C.

11. The method of any one of claims 1 to 10, wherein the cells are selected from the group consisting of stem cells, nerve cells, corneal cells, epithelial cells, and hepatocytes.

12. The method of claim 11, wherein the cells are stem cells.

13. The method of claim 11, wherein the stem cells are mesenchymal stem cells.

14. The method of claim 13, wherein the mesenchymal stem cells are mesenchymal stem cells derived from bone marrow, adipose tissue, placental tissue, synovial tissue, umbilical cord tissue (e.g., cord blood), dental pulp, or amnion, or mesenchymal stem cells differentiated from ES cells or iPS cells.

15. The method of claim 14, wherein the mesenchymal stem cells are bone marrow-derived mesenchymal stem cells.

16. The method of any one of claims 1 to 15, wherein the cells are cells that are used in cell injection therapy and drug discovery research and that are obtained from a subject subjected to the cell injection therapy.

17. The method of claim 16, wherein the platelets are platelets obtained from the subject.

18. A platelet lysate prepared according to the method of any one of claims 1 to 17.

19. A formulation comprising a platelet lysate for the culture of cells obtained from an individual, wherein the platelet lysate is a platelet lysate prepared from platelets obtained from the individual or equivalents thereof, and wherein the platelet lysate has not been inactivated.

20. A method of performing cell infusion therapy in an individual in need thereof, the method comprising the steps of:

   freezing and thawing platelets obtained from the individual or equivalents thereof to obtain a platelet lysate without inactivation;
   culturing cells from the individual in the platelet lysate; and
   injecting an effective amount of the cultured cells into the individual.

Fig. 1

PDGF values according to the number of times of freezing and thawing

[Fig. 1]

EP 4 190 892 A1

31

[Fig. 2]

EP 4 190 892 A1

Fig. 2

Difference in PDGF Extraction Amount by Temperature

-80℃

-20℃

① Thawed Only  ② Thawed + Frozen Once  ③ Thawed + Frozen Twice  ④ Thawed + Frozen Three Times

Fig. 3

BDNF values according to the number of times of freezing and thawing

[Fig. 4]

EP 4 190 892 A1

Fig. 4

Difference in BDNF Extraction Amount by Temperature

[Fig.5]

Fig. 5

TGF-B1 values according to the number of times of freezing and melting

[Fig.6]

Fig. 6

Difference in TGFb1 Extraction Amount by Temperature

Fig. 7

**Difference in PDGF Expression Amount
with or without Decomplementation**

[Fig. 7]

[Fig. 8]

EP 4 190 892 A1

Fig. 8

Difference in BDNF Expression Amount
with or without Decomplementation

[Fig.9]

Fig. 9

Difference in TGF-β1 Expression Amount with or without Decomplementation

[Fig.10]

EP 4 190 892 A1

40

# Fig. 10

|  | Step 1 bone marrow fluid ~P1 | Step 2 P1~P2 | Step 3 P2~P3 | The Whole Total |

[Fig.11]

[Fig.12]

[Fig.13]

Fig. 13

Left) With Decomplementation    Right) Without Decomplementation

EP 4 190 892 A1

[Fig. 14]

Fig. 14

[Fig. 15]

EP 4 190 892 A1

Fig. 15

Fig. 16

TGF-β1 ng/ml

EP 4 190 892 A1

[Fig. 16]

[Fig. 17]

EP 4 190 892 A1

Fig. 17

Difference due to Thawing Temperature after Freezing at -80°C

platelet a

platelet b

■ PDGF-BB  □ T-BDNF  ▨ TGF-β1

[Fig. 18]

EP 4 190 892 A1

Fig.18

PDGF-BB

platelet a

platelet b

Fig. 19

T-BDNF

platelet a

platelet b

[Fig.20]

Fig. 20

TGF-β1

platelet a

platelet b

[Fig. 21]

EP 4 190 892 A1

Fig. 21

**Amount of Growth Factor by PL Production Method**

■ PDGF-BB  □ T-BDNF  ▨ TGF-β1

[Fig.22]

[Fig. 23]

## Fig. 23

## Experiment ③ Culture Result

Growth Curve by PL

Viability AVE

| Culture Days | Day 0 | Day 7 |
|---|---|---|
| -80°C frozen-clinical trial method PL | 100 | 94.2 |
| -80°C frozen - new method PL | 100 | 92.0 |
| -196°C frozen - new method PL | 100 | 90.8 |

(%)

921.9  -196°C frozen - new method PL
771.3  -80°C frozen - new method PL
529.5  -80°C frozen-clinical trial method PL

[Fig. 24]

EP 4 190 892 A1

Fig. 24

[Fig.25]

Fig. 25

[Fig.26]

# Fig. 26

Amount of Exosomes per Cell

[Fig.27]

Fig. 27

## INTERNATIONAL SEARCH REPORT

| International application No. |
|---|
| **PCT/JP2021/028298** |

**A. CLASSIFICATION OF SUBJECT MATTER**

*C12N 5/07*(2010.01)i; *A61K 35/19*(2015.01)i; *A61K 35/28*(2015.01)i; *A61K 35/30*(2015.01)i; *A61K 35/36*(2015.01)i; *A61K 35/407*(2015.01)i; *A61K 35/545*(2015.01)i; *A61P 9/10*(2006.01)i; *A61P 43/00*(2006.01)i; *C12N 5/0735*(2010.01)i; *C12N 5/0775*(2010.01)i; *C12N 5/0793*(2010.01)i

FI: C12N5/07; A61K35/19 Z; A61K35/28; A61K35/30; A61K35/36; A61K35/407; A61K35/545; A61P9/10; A61P43/00 105; C12N5/0735; C12N5/0775; C12N5/0793

According to International Patent Classification (IPC) or to both national classification and IPC

**B. FIELDS SEARCHED**

Minimum documentation searched (classification system followed by classification symbols)

C12N5/07; A61K35/19; A61K35/28; A61K35/30; A61K35/36; A61K35/407; A61K35/545; A61P9/10; A61P43/00; C12N5/0735; C12N5/0775; C12N5/0793

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2011-529706 A (ALLOCURE INC.) 15 December 2011 (2011-12-15) particularly, claims 1-21, paragraphs [0019], [0027], [0050], example 1 | 1-3, 6-20 |
| Y | particularly, claims 1-21, paragraphs [0019], [0027], [0050], example 1 | 4, 5 |
| Y | JP 2018-517771 A (JADI CELL LLC) 05 July 2018 (2018-07-05) particularly, claims 1-56, paragraphs [0015], [0030]-[0034], example 1 | 4, 5 |
| A | particularly, claims 1-56, paragraphs [0015], [0030]-[0034], example 1 | 1-3, 6-20 |
| Y | JP 2016-529283 A (COOK GENERAL BIOTECHNOLOGY LLC) 23 September 2016 (2016-09-23) particularly, paragraphs [0005]-[0019] | 4, 5 |
| A | particularly, paragraphs [0005]-[0019] | 1-3, 6-20 |

☑ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **29 September 2021** | **12 October 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

International application No.

**PCT/JP2021/028298**

**C.    DOCUMENTS CONSIDERED TO BE RELEVANT**

| Category* | Citation of document, with indication, where appropriate, of the relevant passages | Relevant to claim No. |
|---|---|---|
| Y | JP 2014-522833 A (EMORY UNIV.) 08 September 2014 (2014-09-08)<br>particularly, claims 1-15, paragraph [0013], example 1 | 4, 5 |
| A | particularly, claims 1-15, paragraph [0013], example 1 | 1-3, 6-20 |
| A | JP 2014-533715 A (CELL THERAPY LTD.) 15 December 2014 (2014-12-15)<br>entire text | 1-20 |
| A | JP 2019-513154 A (CENTRE HOSPITALIER REGIONAL ET UNIV. DE LILLE) 23 May 2019 (2019-05-23)<br>entire text | 1-20 |
| A | JP 2017-501740 A (ISOPOGEN PTY LTD.) 19 January 2017 (2017-01-19)<br>entire text | 1-20 |

Form PCT/ISA/210 (second sheet) (January 2015)

## INTERNATIONAL SEARCH REPORT
### Information on patent family members

| International application No. |
| --- |
| **PCT/JP2021/028298** |

| Patent document cited in search report | | | Publication date (day/month/year) | Patent family member(s) | | | Publication date (day/month/year) |
| --- | --- | --- | --- | --- | --- | --- | --- |
| JP | 2011-529706 | A | 15 December 2011 | US | 2011/0293576 | A1 | |
| | | | | particularly, claims 1-21, paragraphs [0026], [0035], [0060], example 1 | | | |
| | | | | WO | 2010/017216 | A2 | |
| | | | | EP | 2321408 | A2 | |
| JP | 2018-517771 | A | 05 July 2018 | US | 2018/0163172 | A1 | |
| | | | | particularly, claims 1-56, paragraphs [0015], [0030]-[0033], example 1 | | | |
| | | | | WO | 2016/196515 | A1 | |
| | | | | EP | 3302708 | A1 | |
| JP | 2016-529283 | A | 23 September 2016 | US | 2015/0064133 | A1 | |
| | | | | particularly, paragraphs [0007]-[0010], [0036]-[0040] | | | |
| | | | | WO | 2015/031465 | A1 | |
| | | | | EP | 3038628 | A1 | |
| | | | | CN | 105636599 | A | |
| JP | 2014-522833 | A | 08 September 2014 | US | 2014/0127314 | A1 | |
| | | | | particularly, claims 1-15, paragraph [0013], example 1 | | | |
| | | | | WO | 2013/003356 | A1 | |
| | | | | EP | 2723354 | A1 | |
| | | | | CN | 103702674 | A | |
| JP | 2014-533715 | A | 15 December 2014 | US | 2014/0335195 | A1 | |
| | | | | entire text | | | |
| | | | | WO | 2013/076507 | A2 | |
| | | | | EP | 2782555 | A2 | |
| JP | 2019-513154 | A | 23 May 2019 | US | 2019/0099448 | A1 | |
| | | | | entire text | | | |
| | | | | WO | 2017/162830 | A1 | |
| | | | | EP | 3432895 | A1 | |
| | | | | CN | 109310711 | A | |
| JP | 2017-501740 | A | 19 January 2017 | US | 2015/0307844 | A1 | |
| | | | | entire text | | | |
| | | | | WO | 2015/061839 | A1 | |
| | | | | EP | 3066193 | A1 | |
| | | | | CN | 105765059 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020130744 A

**Non-patent literature cited in the description**

- **KAWABORI et al.** Research on advanced intervention using novel bone marrow stem cell (RAINBOW): a study protocol for a phase I, open-label, uncontrolled, dose-response trial of autologous bone marrow stromal cell transplantation in patients with acute ischemic stroke. *BMC Neurol.,* 08 September 2017, vol. 17 (1), 179

- **RAUCH et al.** *ALTEX,* 2011, vol. 28 (4), 305-16